(19) Europäisches Patentamt
European Patent Office
Office européen des brevets

(11) **EP 3 646 780 A1**

(12) **EUROPEAN PATENT APPLICATION**
published in accordance with Art. 153(4) EPC

(43) Date of publication:
**06.05.2020 Bulletin 2020/19**

(51) Int Cl.:
*A61B 5/02* (2006.01)          *H04R 1/00* (2006.01)
*H04R 17/00* (2006.01)

(21) Application number: **18823464.5**

(22) Date of filing: **04.06.2018**

(86) International application number:
**PCT/JP2018/021388**

(87) International publication number:
**WO 2019/003811 (03.01.2019 Gazette 2019/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.06.2017  JP 2017125505**
         **14.07.2017  JP 2017138398**
         **04.08.2017  JP 2017151907**
         **21.09.2017  JP 2017181820**

(71) Applicant: **Kyocera Corporation**
**Kyoto-shi, Kyoto 612-8501 (JP)**

(72) Inventors:
• **HIRANO Asao**
  **Kyoto-shi**
  **Kyoto 612-8501 (JP)**
• **HIGUCHI Takeshi**
  **Kyoto-shi**
  **Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **ELECTRONIC INSTRUMENT, SERVER, DATA STRUCTURE, PHYSICAL CONDITION MANAGEMENT METHOD, AND PHYSICAL CONDITION MANAGEMENT PROGRAM**

(57)     A wearable device includes a measurement unit that measures biological information of a user. A smartphone includes a controller that performs a predetermined preventive action based on the measured biological information and on atmospheric pressure information. The smartphone further includes a first reporting interface that reports the start of measurement of the biological information based on the atmospheric pressure information. The wearable device also includes a vibration unit that provides the user with vibration as the preventive action.

*FIG. 1*

**Description**

CROSS-REFERENCE TO RELATED APPLICATIONS

The present application claims priority to and the benefit of Japanese Patent Application No. 2017-125505 filed June 27, 2017, Japanese Patent Application No. 2017-138398 filed July 14, 2017, Japanese Patent Application No. 2017-151907 filed August 4, 2017, and Japanese Patent Application No. 2017-181820 filed September 21, 2017, the entire contents of which are incorporated herein by reference.

TECHNICAL FIELD

The present disclosure relates to an electronic device, a server, a data structure, a physical condition management method, and a physical condition management program capable of preventing a change in physical condition due to the weather.

BACKGROUND

An audio device provided with a living body sensing function has been developed. For example, see patent literature (PTL) 1.
It is also known that one's physical condition may worsen for reasons such as a change in atmospheric pressure when the weather changes. A physical condition management application that is usable on a smartphone or the like has been developed focusing on pain, such as headaches, occurring when the atmospheric pressure varies. For example, see non-patent literature (NPL) 1.

CITATION LIST

Patent Literature

PTL 1: JP2016-55155A

Non-patent Literature

NPL 1: SATO, Jun, "Heal weather-related pain and say goodbye to headaches, vertigo, and stress!", 1st edition, 2nd printing, Fusosha Publishing Inc., Nov. 1, 2015, pp. 21-23, pp. 33-37, pp. 112-114

SUMMARY

An electronic device of the present disclosure includes a measurement unit configured to measure biological information of a user and a controller configured to perform a predetermined preventive action based on the biological information measured by the measurement unit and atmospheric pressure information.
An electronic device of the present disclosure includes an environment measurement unit configured to measure environment information of an environment around a user and a controller configured to perform a predetermined preventive action based on the environment information and atmospheric pressure information.
A server of the present disclosure is connected over a network to an electronic device, receives the atmospheric pressure information from the electronic device, and provides the electronic device with a notification of timing for measurement of biological information based on the atmospheric pressure information. The electronic device is configured to report measurement of biological information based on the notification.
A server of the present disclosure is connected over a network to an electronic device, receives the biological information from the electronic device, and transmits an instruction to execute the preventive action to the electronic device based on the biological information. The electronic device is configured to perform the preventive action based on the instruction.
A data structure of the present disclosure includes biological information related to a heart rate of a user and information related to atmospheric pressure. The data structure is configured to cause an electronic device to acquire the information related to the heart rate of the user based on the information related to atmospheric pressure and perform a predetermined preventive action based on the information related to the heart rate.
A physical condition management method of the present disclosure includes measuring biological information of a user and performing a predetermined preventive action based on the measured biological information and atmospheric pressure information.

A physical condition management program of the present disclosure causes a computer to measure biological information of a user and perform a predetermined preventive action based on the measured biological information and atmospheric pressure information.

BRIEF DESCRIPTION OF THE DRAWINGS

In the accompanying drawings:

FIG. 1 is an overall schematic view of a physical condition management system 1000 to which a first embodiment of an electronic device according to the present disclosure is applied;
FIG. 2 is a schematic view of a wearable device 101 illustrated in FIG. 1;
FIG. 3 is a wearing schematic view of the wearable device 101 illustrated in FIG. 1;
FIG. 4 is a schematic configuration diagram of a measurement unit 201L;
FIG. 5 illustrates the internal configuration of each apparatus in the physical condition management system 1000 illustrated in FIG. 1;
FIG. 6 is a table, stored in a storage 507 illustrated in FIG. 5, of atmospheric pressure information by hour at a predetermined location A;
FIG. 7 is graph of the table illustrated in FIG. 6;
FIG. 8 is a conceptual diagram of information stored in the storage 507 illustrated in FIG. 5;
FIG. 9 is a conceptual diagram of a data structure of the information stored in the storage 507;
FIG. 10 is a conceptual diagram of a data structure of the information stored in the storage 507;
FIG. 11 is a conceptual diagram of information stored in a storage 527 illustrated in FIG. 5;
FIG. 12 is a flowchart of operations of the physical condition management system 1000 illustrated in FIG. 1;
FIG. 13 is a graph illustrating atmospheric pressure analysis operations by a smartphone 103 illustrated in FIG. 12;
FIG. 14 is a notification screen, at the start of biological information measurement, displayed on a touch panel display 1001 of a reporting interface 511 of the smartphone 103;
FIG. 15 is a flowchart for the smartphone 103 to judge necessity of a preventive action as illustrated in step S827 of FIG. 12;
FIG. 16 is a flowchart for the smartphone 103 to judge necessity of a preventive action as illustrated in step S827 of FIG. 12;
FIG. 17 is a flowchart for the smartphone 103 to judge necessity of a preventive action as illustrated in step S827 of FIG. 12;
FIG. 18 is a schematic view of a screen, displayed on the touch panel display 1001 of the smartphone 103, instructing to start a preventive action;
FIG. 19 is a schematic view of a massage action reporting screen displayed on a touch panel display of the reporting interface 511 of the smartphone 103;
FIG. 20 is a block diagram illustrating the configuration of a vibration unit 205L;
FIG. 21 is a schematic view illustrating flexing of a panel 1603 due to a piezoelectric element 1601;
FIG. 22 is a schematic configuration diagram of a measurement unit 1801L of an electronic device according to a second embodiment;
FIG. 23 is a schematic view illustrating an example blood flow waveform acquired by the measurement unit 1801L;
FIG. 24 is an internal block diagram of a physical condition management system 20000 that uses a wearable system 2001, which is an electronic device of a third embodiment;
FIG. 25 is a graph illustrating atmospheric pressure analysis operations in an electronic device of a fourth embodiment;
FIG. 26 is a schematic view of a smartphone 2201 used in an electronic device of a fifth embodiment;
FIG. 27 is a side schematic view of the smartphone illustrated in FIG. 26;
FIG. 28 is an internal block diagram of a physical condition management system 24000 using the smartphone 2201 illustrated in FIG. 26;
FIG. 29 is a flowchart of operations of the physical condition management system 24000 illustrated in FIG. 28;
FIG. 30 is a schematic configuration diagram of a wearable system 2601, which is an electronic device of a sixth embodiment;
FIG. 31 is a schematic configuration diagram of a left-ear earphone 2603L illustrated in FIG. 30;
FIG. 32 is a flowchart of operations by a seventh embodiment of an electronic device of the present disclosure;
FIG. 33 is a table illustrating the relationship between frequency and power for blood flow obtained with a blood flow meter;
FIG. 34 is graph of the table illustrated in FIG. 33;
FIG. 35 is a block diagram of a physical condition management system 35000 that uses an electronic device of an eighth embodiment;

FIG. 36 is a flowchart of operations of the physical condition management system 35000 illustrated in FIG. 35;

FIG. 37 is an internal block diagram of a physical condition management system 37000 that uses a wearable system 3700, which is an electronic device of a ninth embodiment;

FIG. 38 is a schematic configuration diagram of a measurement unit 3711L;

FIG. 39 is a schematic view of a screen, displayed on the touch panel display 1001 of the smartphone 103, instructing to start an action to raise body temperature;

FIG. 40 is a schematic view of a screen, displayed on the touch panel display 1001 of the smartphone 103, instructing to start an action to lower body temperature;

FIG. 41 is an internal block diagram of a physical condition management system 41000 that uses a wearable system 4100, which is an electronic device of a tenth embodiment;

FIG. 42 is a schematic configuration diagram illustrating the case of the measurement unit 201L including a temperature sensor 4111 and a humidity sensor 4113;

FIG. 43 is a flowchart of operations of the physical condition management system 41000 illustrated in FIG. 41;

FIG. 44 is a schematic view of a first preventive action;

FIG. 45 is a schematic view of the first preventive action;

FIG. 46 is a flowchart of the first preventive action;

FIG. 47 is a flowchart of the first preventive action;

FIG. 48 is a conceptual diagram of transitions between combinations of music and vibration during the first preventive action; and

FIG. 49 is a schematic operation view of a second preventive action.

DETAILED DESCRIPTION

First Embodiment

A first embodiment of an electronic device according to the present disclosure is described below with reference to the drawings. The following description of embodiments of an electronic device according to the present disclosure also serves to describe embodiments of a server, a data structure, a physical condition management method, and a physical condition management program according to the present disclosure.

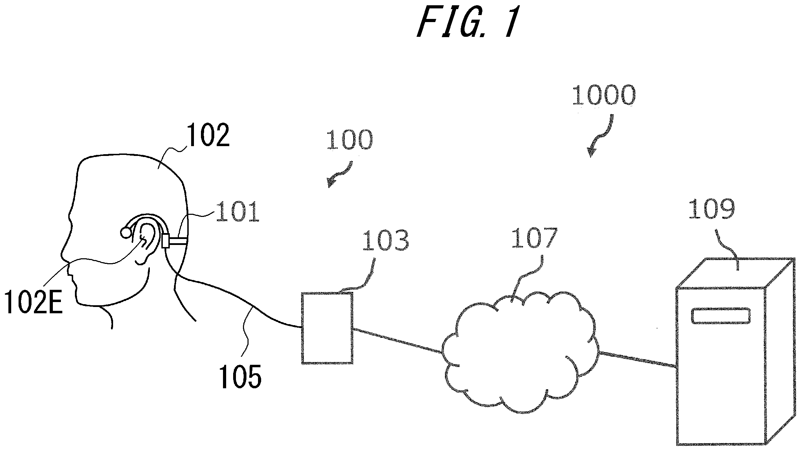

FIG. 1 is an overall schematic view of a physical condition management system 1000 to which a first embodiment of an electronic device according to the present disclosure is applied. As illustrated in FIG. 1, the physical condition management system 1000 includes a wearable system 100, which is the first embodiment of an electronic device according to the present disclosure, and a server 109 connected to the wearable system 100 over a network 107.

The wearable system 100 includes a wearable device 101 that is worn on the ears 102E of a user 102 and a smartphone 103 that connects to the wearable device 101 over a cable 105. The wearable device 101 and the smartphone 103 perform at least one of transmission and reception of information over the cable 105. This smartphone 103 is the main unit of the wearable device 101.

The wearable device 101 and the smartphone 103 have been described as being connected over a wired cable 105. In the present disclosure, however, the wearable device 101 and the smartphone 103 may perform at least one of transmission and reception of information over a wireless connection instead of or in addition to the wired connection.

The smartphone 103 and the server 109 are connected over the network 107. The network 107 may be wired, wireless, or a combination of both. The network 107 may be any combination of the Internet, a wireless LAN, a wired LAN, a public telephone network, or the like. In FIG. 1, a solid line is depicted between the smartphone 103 and the network 107, and between the server 109 and the network 107, to indicate conceptually that the network 107 is connected to the smartphone 103 and the server 109. The connection between the network 107 and the smartphone 103 and server 109 may be wired, wireless, or a combination thereof.

The number of smartphones in the present disclosure is not limited to one and may be any number equal to or greater than one. Furthermore, the number of servers in the present disclosure is not limited to one and may be any number equal to or greater than one. In the present disclosure, a plurality of servers may execute the same or different functions.

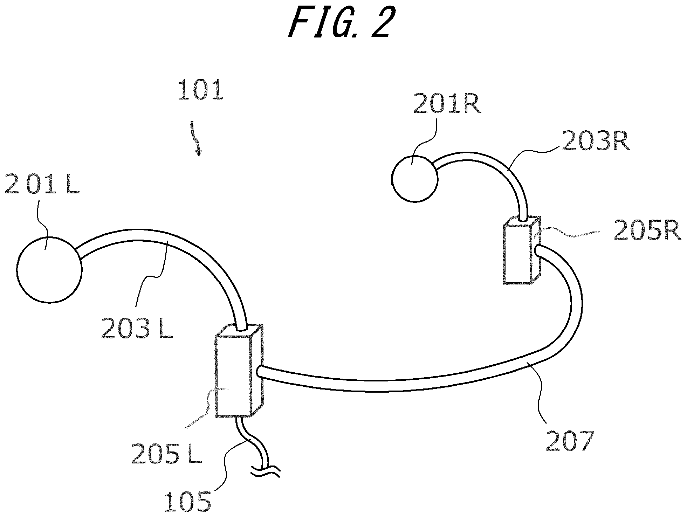

Next, the wearable device 101 illustrated in FIG. 1 is described with reference to FIG. 2. FIG. 2 is a schematic view of the wearable device 101 illustrated in FIG. 1.

As illustrated in FIG. 2, the wearable device 101 includes a holding portion 203L held by the auricle of the left ear of a user. The wearable device 101 also includes a vibration unit 205L provided in the holding portion 203L at the back head side of the left ear of the user. The wearable device 101 also includes a measurement unit 201L provided in the holding portion 203L at the face side of the left ear of the user. The vibration unit 205L includes the cable 105 connected to the smartphone 103. The cable 105 may be provided at a location other than the vibration unit 205L.

As illustrated in FIG. 2, the wearable device 101 includes a holding portion 203R held by the auricle of the right ear of the user. The wearable device 101 also includes a vibration unit 205R provided in the holding portion 203R at the back head side of the right ear of the user. The wearable device 101 also includes a measurement unit 201R provided in the holding portion 203R at the face side of the right ear of the user. At least one of the measurement units 201L, 201R is, for example, disposed in the temporal region. At least one of the measurement units 201L, 201R may be disposed in an area other than the temporal region.

The wearable device 101 illustrated in FIG. 2 includes a connector 207 connecting the vibration units 205L, 205R. Plastic, rubber, cloth, paper, resin, iron, another material, or any combination thereof may be used in the holding portion 203L, the holding portion 203R, and the connector 207. At least one of the vibration units 205L, 205R is, for example, disposed at the mastoid process. At least one of the vibration units 205L, 205R may, for example, be disposed at a location other than the mastoid process.

Vibration by at least one of the vibration units 205L, 205R is, for example, preferably 7 Hz or greater. Vibration by at least one of the vibration units 205L, 205R can stimulate the vestibular nerve in the ear. Vibration by at least one of the vibration units 205L, 205R may be less than 7 Hz. Vibration by at least one of the vibration units 205L, 205R may be equal to or greater than 30 Hz.

The wearable device 101 of the present disclosure may, for example, be configured by omitting at least one of the measurement unit 201R and the vibration unit 205R from the configuration illustrated in FIG. 2. The wearable device 101 of the present disclosure may, for example, be configured by omitting at least one of the measurement unit 201L and the vibration unit 205L from the configuration illustrated in FIG. 2. The wearable device 101 of the present disclosure may, for example, be configured by omitting the measurement unit 201R and the vibration unit 205L from the configuration illustrated in FIG. 2. The wearable device 101 of the present disclosure may, for example, be configured by omitting the measurement unit 201L and the vibration unit 205R from the configuration illustrated in FIG. 2.

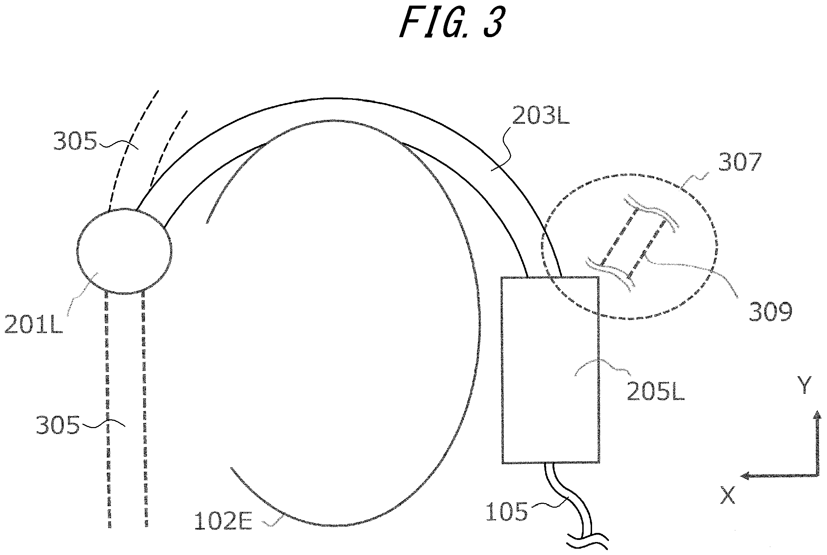

Next, the case of the wearable device 101 illustrated in FIG. 1 being worn by a user is described with reference to FIG. 3. FIG. 3 is a wearing schematic view of the wearable device 101 illustrated in FIG. 1. In FIG. 3, the X-axis direction is the direction faced by the front of the user's face, and the Y-axis direction is the direction from the user's jaw towards the top of the head. In FIG. 3, the state in which the user is wearing the wearable device 101 on the left ear is illustrated, but the state on the right ear of the user is similar to FIG. 3.

As illustrated in FIG. 3, the holding portion 203L is held near the top of the user's left ear 102E. The measurement unit 201L is on the holding portion 203L. The measurement unit 201L is in contact with the user's skin surface. A superficial temporal artery 305 is on the inside of the user's skin. The holding portion 203L is disposed at a position covering the superficial temporal artery 305 from above the user's skin.

As illustrated in FIG. 3, the vibration unit 205L is disposed at the posterior auricle of the user's left ear 102E. The vibration unit 205L is in contact with the user's skin surface. A vestibular nerve 309 is located near the inner ear in an internal region 307 below the skin surface contacted by the vibration unit 205L.

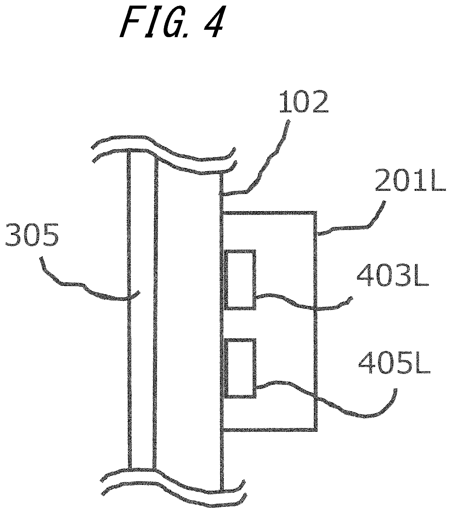

Next, the configuration of the measurement unit 201L is described with reference to FIG. 4. FIG. 4 is a schematic configuration diagram of the measurement unit 201L. The configuration of the measurement unit 201R illustrated in FIG. 2 is similar to the configuration illustrated in FIG. 4.

The measurement unit 201L is a plethysmograph. The measurement unit 201L includes an optical emitter 403L and an optical detector 405L. The measurement unit 201L measures the change in blood volume of at least one of an artery and a capillary, which corresponds to the change in heart rate, to obtain information of a pulse wave as information related to the heart rate. A plethysmogram is the result of treating the expansion and contraction of a blood vessel as a waveform. A plethysmograph detects the change in light absorption based on the change in volume of a blood vessel by emitting light from an optical emitter and detecting the light with an optical detector, thereby detecting a pulse wave.

The optical emitter 403L may, for example, be a light emitting diode (LED). The optical detector 405L may, for example, be a photodiode (PD). The wavelength of light emitted by the optical emitter 403L may be infrared light of 800 nm or greater. The wavelength of light emitted by the optical emitter 403L may be visible light of 380 nm or greater and less than 800 nm. Furthermore, light having a wavelength near the ranges of 435 nm to less than 480 nm, 500 nm to less than 560 nm, and 610 nm to less than 750 nm, or light of a different wavelength, may also be suitably used as the light emitted by the optical emitter 403L.

The light emitted by the optical emitter 403L is scattered at the superficial temporal artery 305 and is then incident on the optical detector 405L.

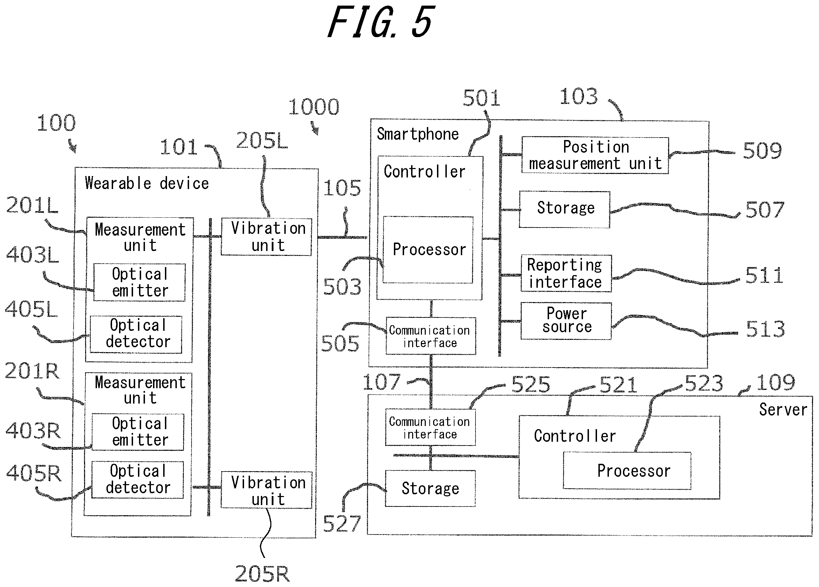

Next, with reference to FIG. 5, the internal configuration of the physical condition management system 1000 illustrated in FIG. 1 is described.

FIG. 5 illustrates the internal configuration of each apparatus in the physical condition management system 1000 illustrated in FIG. 1.

As illustrated in FIG. 5, the physical condition management system 1000 includes the wearable device 101, the smartphone 103 connected to the wearable device 101 by the cable 105, and the server 109. The smartphone 103 functions as the main unit.

The wearable device 101 includes the measurement unit 201R, the measurement unit 201L, the vibration unit 205R, and the vibration unit 205L as functional blocks.

The measurement unit 201R includes an optical emitter 403R and an optical detector 405R. The measurement unit 201L includes the optical emitter 403L and the optical detector 405L.

Operations of the measurement unit 201R, the measurement unit 201L, the vibration unit 205R, and the vibration unit 205L are controlled by a controller 501.

Next, the internal configuration of the smartphone 103 is described with reference to FIG. 5. The smartphone 103 includes the controller 501, a communication interface 505, a storage 507, a position measurement unit 509, a reporting interface 511, and a power source 513.

The controller 501 is a processor that controls and manages the entire wearable system 100, such as the components of the wearable device 101, as well as the functional blocks of the smartphone 103. The controller 501 is a processor, such as a central processing unit (CPU), that executes programs with prescribed control procedures. Such programs may, for example, be stored in the storage 507 or on an external storage medium or the like connected to the smartphone 103.

To provide control and processing capability for executing various functions, as described below in greater detail, the smartphone 103 includes at least one processor 503.

In various embodiments, the one or more processors 503 may be implemented as a single integrated circuit (IC) or as a plurality of communicatively connected integrated circuits and/or discrete circuits. The one or more processors 503 can be implemented with a variety of known techniques.

In an embodiment, the processor 503 includes one or more circuits or units configured to execute one or more data calculation procedures or processes by executing instructions stored in related memory, for example. In another embodiment, the processor 503 may be firmware (such as discrete logic components) configured to execute one or more data calculation procedures or processes.

In various embodiments, the processor 503 may include one or more processors, controllers, microprocessors, microcontrollers, application specific integrated circuits (ASIC), digital signal processors, programmable logic devices, field programmable gate arrays, any combination of these devices or structures, or a combination of other known devices and structures, to execute the below-described functions of the controller 501.

Based on atmospheric pressure information stored in the storage 507, for example, the controller 501 causes the reporting interface 511 to report the start of biological information measurement. Based on the instruction from the controller 501, the reporting interface 511 uses audio, an image, vibration, or a combination of these to report the start of measurement of biological information. In other words, the reporting interface 511 has the function of a first reporting interface.

The communication interface 505 transmits and receives various information by communicating with a communication interface 525 of the server 109 over the network 107 by wired communication, wireless communication, or a combination of wired and wireless communication. For example, the communication interface 505 transmits information related to the plethysmogram of the user measured by the measurement units 201L, 201R to the server 109.

The storage 507 can be configured by a semiconductor memory, a magnetic memory, or the like. The storage 507 stores various information, programs for operating the smartphone 103 and the wearable device 101, and the like. The storage 507 may also function as a working memory. For example, the storage 507 may store received information related to the atmospheric pressure.

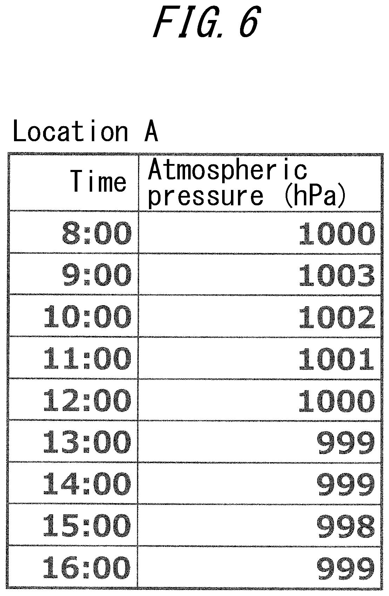

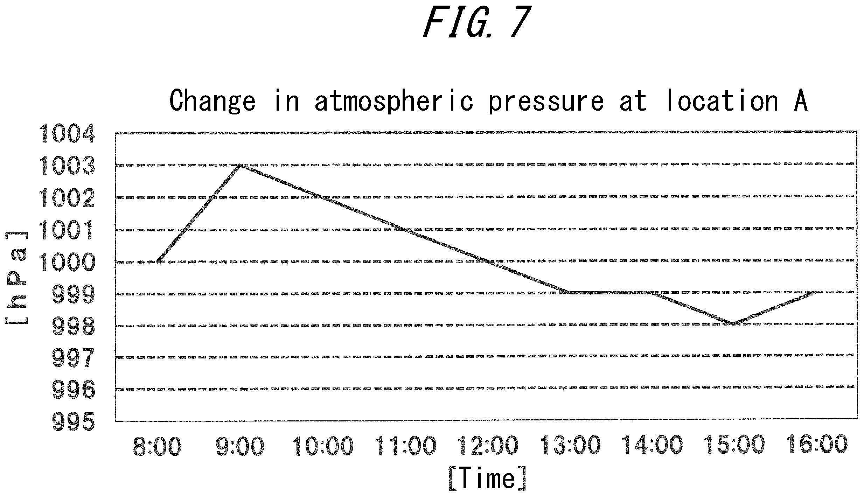

Here, the atmospheric pressure information stored in the storage 507 is described with reference to FIG. 6 and FIG. 7. FIG. 6 is a table, stored in the storage 507 illustrated in FIG. 5, of atmospheric pressure information by hour at a predetermined location A. Only nine atmospheric pressures from 8:00 to 16:00 are illustrated in the example in FIG. 6, but the number of atmospheric pressures from 8:00 to 16:00 in the present disclosure is not limited to nine and may be any number in any time range. Furthermore, while only one location A is illustrated, the number of predetermined locations for which atmospheric pressure information is stored in the storage 507 in the present disclosure may be any number one or greater. Atmospheric pressure information by hour is illustrated in the example in FIG. 6, but in the present disclosure, the atmospheric pressure information may be indicated at any time interval other than one hour, such as every minute, every 30 minutes, or every three hours. These time intervals need not be constant. Atmospheric pressure information may be stored at different time intervals in the present disclosure. The atmospheric pressure information in FIG. 6 is indicated as a whole number, but the storage 507 may store digits after the decimal point in the present disclosure.

The atmospheric pressure information stored in the storage 507 may be information received from the server 109, for example, over the network 107. The atmospheric pressure information stored in the storage 507 may be information inputted by the user, for example.

As illustrated in FIG. 6, the atmospheric pressure at location A is 1000 [hPa] at time 8:00, 1003 [hPa] at time 9:00, 1002 [hPa] at time 10:00, 1001 [hPa] at time 11:00, 1000 [hPa] at time 12:00, 999 [hPa] at time 13:00, 999 [hPa] at time 14:00, 998 [hPa] at time 15:00, and 999 [hPa] at time 16:00.

FIG. 7 is a graph of the table in FIG. 6. In FIG. 7, the horizontal axis represents time, and the vertical axis represents atmospheric pressure [hPa].

The position measurement unit 509 measures the current position of the smartphone. This position measurement may be made using a global positioning system (GPS), for example. The position information measured by the position measurement unit 509 is stored in the storage 507.

The reporting interface 511 provides a predetermined report. This report is an image, audio, light emission, vibration, or a combination of these. The reporting interface 511 may be a touch panel display, a liquid crystal display, a speaker, an LED, a vibration motor, or any combination of these devices. The reporting interface 511 may provide any report other than the ones described above. The reporting interface 511 may include a device other than the above devices.

In other words, the reporting interface 511 may report information by sound, vibration, images, and the like. The reporting interface 511 may include a speaker, a vibration unit, and a display device. The display device can, for example, be a liquid crystal display (LCD), an organic electro-luminescence display (OELD), an inorganic electro-luminescence display (IELD), or the like. The reporting interface 511 may, for example, report the measurement timing of biological information, information for physical condition management, and the like.

The wearable device 101 may be configured to measure biological information automatically when being worn by the user, without an instruction from the user. The wearable device 101 may, for example, use a living body detection sensor, such as an illuminance sensor, to detect wearing by the user. The wearable device 101 may, for example, start measurement of biological information based on a measurement instruction signal transmitted from the smartphone 103. When the atmospheric pressure information changes, the smartphone 103 may transmit the measurement instruction signal to the wearable device 101 in response to a user instruction or without a user instruction.

The power source 513 provides power to the smartphone 103 and the wearable device 101. A lithium battery, which is a secondary cell, or the like can be used as the power source 513, for example.

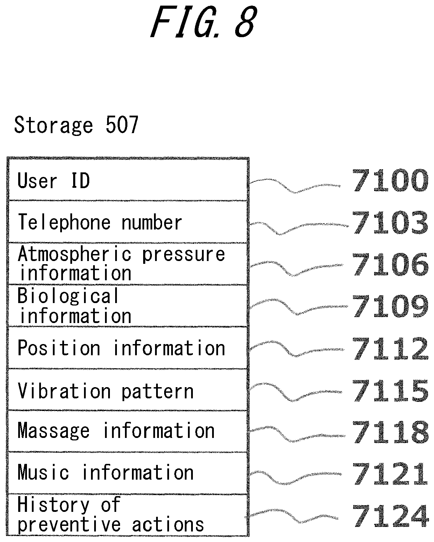

Next, the information stored in the storage 507 is described with reference to FIG. 8. FIG. 8 is a conceptual diagram of information stored in the storage 507 illustrated in FIG. 5. The information illustrated in FIG. 8 is only an example of information stored in the storage 507. Other information may be stored in the storage 507, and a portion of the information illustrated in FIG. 8 may be removed. Other than the information illustrated in FIG. 8, various control programs, application programs, and the like may also be stored in the storage 507.

A user ID 7100 in FIG. 8 is information for identifying the user. There may be one or more user IDs 7100. A telephone number 7103 is the telephone number of the smartphone 103. There may be one or more telephone numbers 7103.

Atmospheric pressure information 7106 is atmospheric pressure information received by the smartphone 103. This atmospheric pressure information 7106 may include atmospheric pressure information not only for the area in which the smartphone 103 is located, but also for other areas. A plurality of pieces of atmospheric pressure information 7106 may be stored at any time intervals.

The biological information 7109 is measured biological information of the user. Examples of this biological information include a plethysmogram, pulse variability, power spectral density of pulse variability, blood pressure, body temperature, blood glucose level, LF/HF value, blood flow, or any combination thereof.

Position information 7112 is a history of the position of the smartphone 103. A vibration pattern 7115 is information of a pattern when the wearable device 101 causes the vibration units 205R, 205L to vibrate. There may be one or more of these patterns.

Massage information 7118 is massage-related information reported to the user. Examples of the massage information 7118 include image-based massage instructions and audio-based massage instructions. There may be one or more massages.

Music information 7121 is information of music to output. There may be one or more types of music. This music may be music that allows the user to relax. This music may, for example, be music at a Solfeggio frequency. The Solfeggio frequencies are, for example, 174 hz, 285 hz, 396 hz, 417 hz, 528 hz, 639 hz, 741 hz, 852 hz, and 963 hz. The types of music indicated by the music information 7172 in the present disclosure are not limited and may include classical music, popular music, jazz music, music with a continuous sound at a constant frequency, music that includes people's voices, instrumental music, music that includes sounds of animals or natural phenomena, or music combining any of these types. Based on the music information 7121, the controller 501 causes the vibration units 205L, 205R to vibrate, thereby outputting audio corresponding to the music information 7121 to the user. In other words, the vibration units 205L, 205R function as an output interface.

A history 7124 of preventive actions is information on the type, date and time, and the like of preventive actions performed on the user. There may be one or more histories of preventive actions.

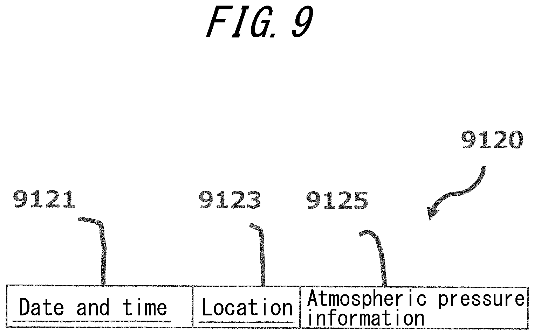

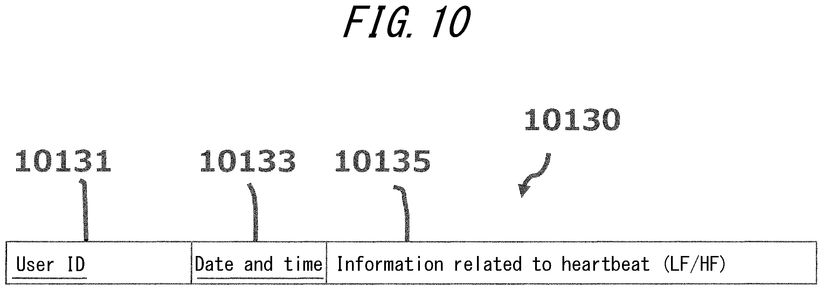

Next, a more detailed data structure of the information stored in the storage 507 is described with reference to FIG. 9 and FIG. 10. FIG. 9 and FIG. 10 are conceptual diagrams of the data structure of information stored in the storage 507.

As illustrated in FIG. 9, a data structure 9120 that includes atmospheric pressure information 9125 as information having a date and time 9121 and location 9123 as a primary key is stored in the storage 507.

The date and time 9121 is a date and time for identifying the atmospheric pressure information 9125. The date and time 9121 includes at least one of the year, month, day, time, and seconds and is represented as any combination thereof. The date and time 9121 may be represented as a predetermined time period.

The location 9123 is a location for identifying the atmospheric pressure information 9125. The location 9123 is represented by an area over a predetermined range, such as a region, country, prefecture, state, local government, building, facility, or a combination thereof.

The atmospheric pressure information 9125 is atmospheric pressure information for the date and time 9121 and the location 9123. The atmospheric pressure information 9125 may be information received from an external source or may be a value measured by the smartphone 103.

As illustrated in FIG. 10, a data structure 10130 that includes information related to the heart rate (LF/HF) 10135 as information having a user ID 10131 and a date and time 10133 as a primary key is stored in the storage 507.

The user ID 10131 is information for identifying the user. The date and time 10133 is similar to the above-described date and time 9121.

The information related to the heart rate 10135 is biological information of the user. The information related to the heart rate 10135 is the LF/HF value at the date and time 10133 based on the values of the LF component and the HF component, which are calculated from heart rate variability extracted from the measured pulse.

The controller 501 of the smartphone 103 performs a preventive action related to the user's physical condition using the above-described data structure 9120 and data structure 10130. Specifically, the controller 501 uses the data structure 9120 to acquire date and time information T1 at the point in time at which the atmospheric pressure at a predetermined location drops.

Subsequently, the controller 501 acquires biological information at the time point T1 and a time point T2 that is a predetermined length of time after the time point T1 and creates the data structure 10130.

The controller 501 then uses the data structure 10130 to compare the value related to LF/HF at time point T1 and the value related to LF/HF at time point T2 and judges whether to have the user perform a preventive action.

The data structure 9120 of FIG. 9 and the data structure 10130 of FIG. 10 have been described as being stored in the storage 507 of the smartphone 103. The data structure 9120 and the data structure 10130 may, however, be stored in a storage 527 of the server 109. A controller 521 of the server may use the data structure 9120 and the data structure 10130 to judge whether to have the user start a preventive action.

The server 109 includes the controller 521, the communication interface 525, and the storage 527.

The controller 521 is a processor that controls and manages the server 109 overall, including the functional blocks of the server 109. The controller 521 is a processor, such as a CPU, that executes a program prescribing control procedures. Such programs may, for example, be stored in the storage 527 or on an external storage medium or the like connected to the server 109.

To provide control and processing capability for executing various functions, as described below in greater detail, the controller 521 includes at least one processor 523.

In various embodiments, the one or more processors 523 may be implemented as a single integrated circuit (IC) or as a plurality of communicatively connected integrated circuits and/or discrete circuits. The one or more processors 523 can be implemented with a variety of known techniques.

In an embodiment, the processor 523 includes one or more circuits or units configured to execute one or more data calculation procedures or processes by executing instructions stored in related memory, for example. In another embodiment, the processor 523 may be firmware (such as discrete logic components) configured to execute one or more data calculation procedures or processes.

In various embodiments, the processor 523 may include one or more processors, controllers, microprocessors, microcontrollers, application specific integrated circuits (ASIC), digital signal processors, programmable logic devices, field programmable gate arrays, any combination of these devices or structures, or a combination of other known devices and structures, to execute the below-described functions of the controller 521.

The storage 527 can be configured by a semiconductor memory, a magnetic memory, or the like. The storage 527 stores various information, programs for operating the server 109, and the like. The storage 527 may also function as a working memory. The storage 527 may, for example, store a program for executing an application to transmit atmospheric pressure information to the smartphone 103.

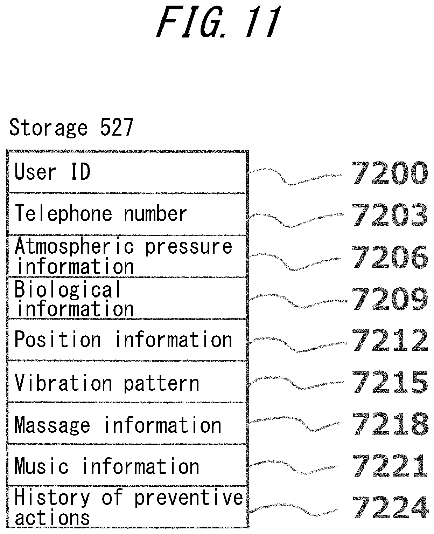

Next, the data stored in the storage 527 is described with reference to FIG. 11. FIG. 11 is a conceptual diagram of information stored in the storage 527 illustrated in FIG. 5. The information illustrated in FIG. 11 is only an example of information stored in the storage 527. Other information may be stored in the storage 527, and a portion of the information illustrated in FIG. 11 may be excluded. Other than the information illustrated in FIG. 11, various control programs, application programs, and the like may also be stored in the storage 527.

Some of the information stored in the storage 527 is received from the smartphone 103. Some of the information stored in the storage 527 is received from another server connected to the server 109 over a network.

A user ID 7200 in FIG. 11 is information for identifying the user of the smartphone 103. There may be one or more user IDs 7200. A telephone number 7203 is the telephone number of the smartphone 103. There may be one or more telephone numbers 7203. The server 109 receives the user ID 7200 and the telephone number 7203 from the smartphone 103.

Atmospheric pressure information 7206 is atmospheric pressure information received by the server 109. This atmospheric pressure information 7206 may include atmospheric pressure information not only for the area in which the server 109 is located, but also for other areas. A plurality of pieces of atmospheric pressure information 7206 may be stored at any time intervals.

Biological information 7209 is biological information of the user and is received from the smartphone 103. Examples of this biological information include a plethysmogram, pulse variability, power spectral density of pulse variability, blood flow, or any combination thereof.

Position information 7212 is a history of the position of the smartphone 103. The server 109 receives the position information 7212 from the smartphone 103. A vibration pattern 7215 is information of a pattern when the smartphone 103 causes the vibration units 205R, 205L of the wearable device 101 to vibrate. There may be one or more of these patterns.

Massage information 7218 is massage-related information reported to the user of the smartphone 103. Examples of the massage information 7218 include image-based massage instructions and audio-based massage instructions. There may be one or more massages. Examples of massages include a massage to move or warm the ear.

Music information 7221 is information of music outputted by the smartphone 103. There may be one or more types of music. This music may be music that allows the user to relax. This music may, for example, be music including sounds at Solfeggio frequencies. Examples of music including sounds at Solfeggio frequencies include music consisting only of sounds at Solfeggio frequencies and music in which sounds at Solfeggio frequencies are combined with any other sounds. When sounds at Solfeggio frequencies are combined with other sounds, the sounds at Solfeggio frequencies should have a sufficient volume to allow the user to distinguish between the other sounds and the sounds at Solfeggio frequencies. The music indicated by the music information 7221 may, for example, be music including many sounds at Solfeggio frequencies. Music including many sounds at Solfeggio frequencies is music in which sounds at Solfeggio frequencies are emphasized. In other words, the volume of the sounds at Solfeggio frequencies is increased in this music so that the sounds at Solfeggio frequencies have a sufficient volume to allow the user to distinguish between other sounds and the sounds at Solfeggio frequencies. The Solfeggio frequencies are, for example, 174 hz, 285 hz, 396 hz, 417 hz, 528 hz, 639 hz, 741 hz, 852 hz, and 963 hz. The types of music indicated by the music information 7221 in the present disclosure are not limited and may include classical music, popular music, jazz music, music with a continuous sound at a constant frequency, music that includes people's voices, instrumental music, music that includes sounds of animals or natural phenomena, or music combining any of these types.

In other words, the smartphone 103 may cause the user to hear sounds at Solfeggio frequencies by playing back one or a plurality of types of music with sounds at Solfeggio frequencies overlapped thereon from a vibration unit or a speaker. Sound at the aforementioned frequencies may be played to the user continuously, or a single sound may be played for the user repeatedly or intermittently. The smartphone 103 may play back a song with many sounds at the aforementioned Solfeggio frequencies from a vibration unit or a speaker.

Based on the music information 7221, the controller 501 causes the vibration units 205L, 205R to vibrate, thereby outputting audio corresponding to the music information 7221 to the user. In other words, the vibration units 205L, 205R function as an output interface.

A history 7224 of preventive actions is information on the type, date and time, and the like of preventive actions performed on the user of the smartphone 103. There may be one or more histories 7224 of preventive actions. The server 109 receives the history 7224 of preventive actions from the smartphone 103.

The communication interface 525 transmits and receives various information by communicating with the smartphone 103 by wired communication, wireless communication, or a combination of wired and wireless communication. For example, the communication interface 525 receives pulse-related information, which is biological information of the user measured by the smartphone 103, from the smartphone 103.

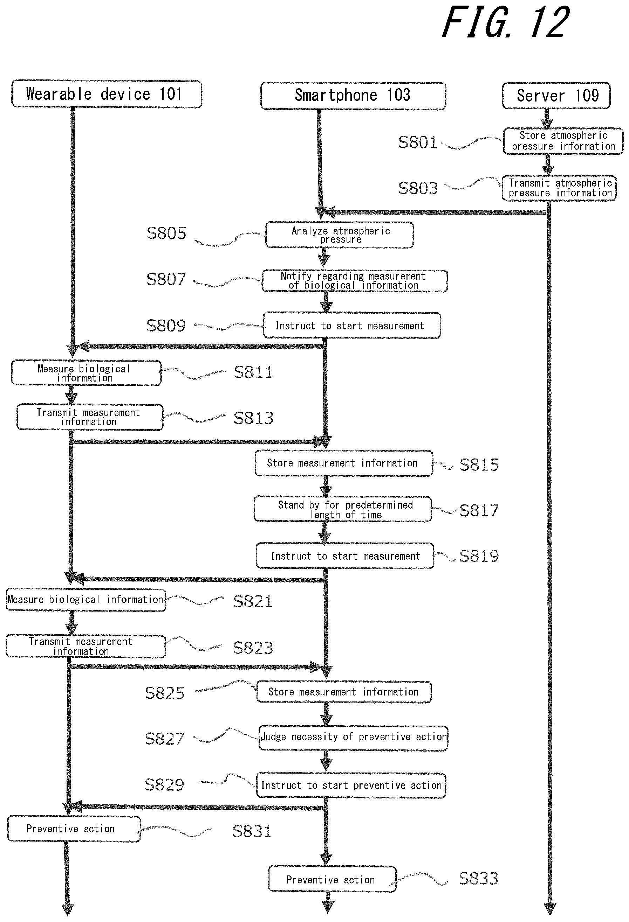

Next, with reference to FIG. 12, the operations of the physical condition management system 1000 illustrated in FIG. 1 are described. FIG. 12 is a flowchart of operations of the physical condition management system 1000 illustrated in FIG. 1.

As illustrated in FIG. 12, the server 109 first stores atmospheric pressure information in the storage 527 (S801). This atmospheric pressure information is, for example, atmospheric pressure information distributed by the government or the like. The atmospheric pressure information may also be atmospheric pressure information created by a private institution. The server 109 transmits the atmospheric pressure information to the smartphone 103 (S803). The server 109 may receive the atmospheric pressure information through the network 107 and store the atmospheric pressure information in the storage 527. The atmospheric pressure information may be stored in the storage 527 by the user inputting the atmospheric pressure information to the server 109.

After receiving the atmospheric pressure information from the server 109, the smartphone 103 stores the received atmospheric pressure information in the storage 507 and analyzes the atmospheric pressure information (S805).

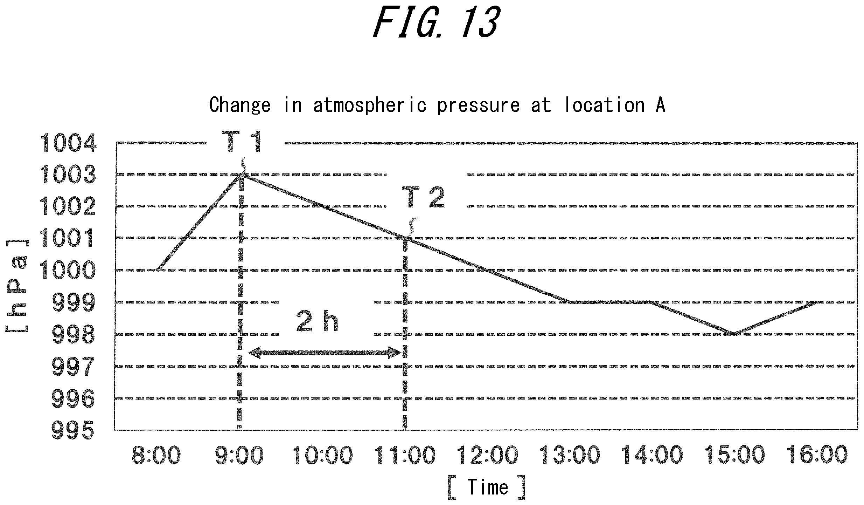

The atmospheric pressure analysis operation in step S805 is now described. Based on the atmospheric pressure information stored in the storage 507, the smartphone 103 identifies the time point T1 at which the atmospheric pressure starts to drop, as illustrated in FIG. 13. The time 9:00 is T1 in the graph in FIG. 13. Here, FIG. 13 is a graph illustrating atmospheric pressure analysis operations by the smartphone 103 illustrated in FIG. 12.

Based on the analysis result of the atmospheric pressure information, the smartphone 103 then provides notification of the start of biological information measurement (S807). Notification of the start of measurement is, for example, provided by the display of an image on a touch panel display, or by audio or vibration. Transmission of this notification may also begin in response to a user-inputted instruction, such as when the notification is transmitted after the user touches an instruction button, displayed on the touch panel display, for starting biological information measurement.

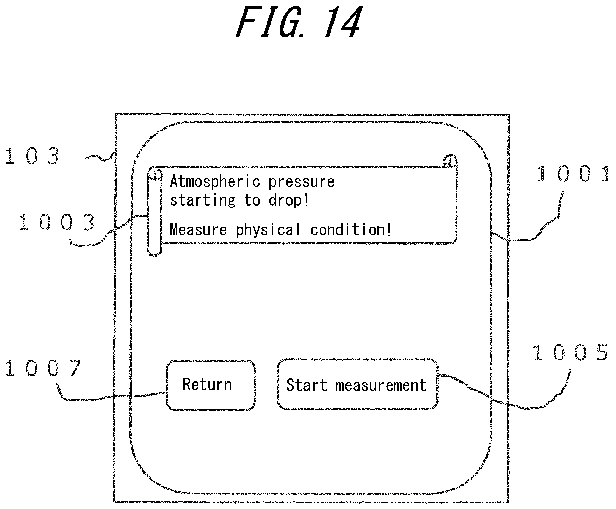

With reference to FIG. 14, the notification of the start of biological information measurement displayed on the smartphone 103 is now described.

FIG. 14 is a notification screen, at the start of biological information measurement, displayed on the touch panel display 1001 serving as the reporting interface 511 of the smartphone 103.

As illustrated in FIG. 14, a message 1003 providing notification of the drop in atmospheric pressure and recommending physical condition measurement, a measurement start button 1005, and a return button 1007 are displayed on the touch panel display 1001 of the smartphone 103.

When the user touches the measurement start button 1005, a control signal for starting measurement of biological information is transmitted to the measurement unit of the wearable device 101, and measurement of biological information starts.

When the user touches the return button 1007, a different image is displayed on the touch panel display 1001.

Based on the analysis result of the atmospheric pressure, the smartphone 103 transmits an instruction to start measurement of biological information to the wearable device 101 (S809). The wearable device 101 may be configured to measure biological information automatically in step S809 when being worn by the user, without an instruction from the user. In this case, the wearable device 101 may, for example, use a living body detection sensor, such as an illuminance sensor, to detect wearing by the user. The wearable device 101 may, for example, start measurement of biological information based on a measurement instruction signal transmitted from the smartphone 103, without user instruction. When the atmospheric pressure changes, the smartphone 103 may transmit the measurement instruction signal to the measurement units 201R, 201L of the wearable device 101 even without a user instruction.

After receiving the instruction to start measurement of biological information from the smartphone 103, the wearable device 101 starts measurement of biological information (S811). The wearable device 101 uses the measurement unit 201R and the measurement unit 201L to measure information indicating a plethysmogram of the user in the present disclosure.

The wearable device 101 may be configured to measure biological information automatically when being worn by the user, without an instruction from the user. The wearable device 101 may, for example, use a living body detection sensor, such as an illuminance sensor, to detect wearing by the user. The wearable device 101 may, for example, start measurement of biological information based on a measurement instruction signal transmitted from the smartphone 103. When the atmospheric pressure information changes, the smartphone 103 may transmit the measurement instruction signal to the wearable device 101 even without a user instruction.

The wearable device 101 transmits the measured biological information to the smartphone 103 (S813).

The smartphone 103 stores the biological information received from the wearable device 101 in the storage 507 (S815).

The smartphone 103 stands by for a predetermined length of time from time point T1 (S817). The smartphone 103 stands by for two hours in step S817. While this predetermined elapsed time is two hours in this example, the predetermined elapsed time may be any length of time other than two hours, such as five minutes, 30 minutes, one hour, six hours, one day, or the like.

After standing by for this predetermined length of time, the smartphone 103 instructs the measurement units 201R, 201L of the wearable device 101 to start measuring biological information (S819). The wearable device 101 may be configured to measure biological information automatically in step S819 when being worn by the user, without an instruction from the user. In this case, the wearable device 101 may, for example, use a living body detection sensor, such as an illuminance sensor, to detect wearing by the user. The wearable device 101 may, for example, start measurement of biological information based on a measurement instruction signal transmitted from the smartphone 103, without user instruction. When the atmospheric pressure information changes, the smartphone 103 may transmit the measurement instruction signal to the measurement units 201R, 201L of the wearable device 101 even without a user instruction.

After receiving the instruction to start measurement of biological information from the smartphone 103, the wearable device 101 starts measurement of biological information (S821). The wearable device 101 measures information indicating a plethysmogram of the user in the present disclosure.

The wearable device 101 transmits the measured biological information to the smartphone 103 (S823).

The smartphone 103 stores the biological information received from the wearable device 101 in the storage 507 (S825).

The smartphone 103 compares two pieces of information related to biological information stored in the storage 507 in S815 and S825. When a predetermined condition is satisfied, the smartphone 103 judges whether a preventive action is to be performed (S827).

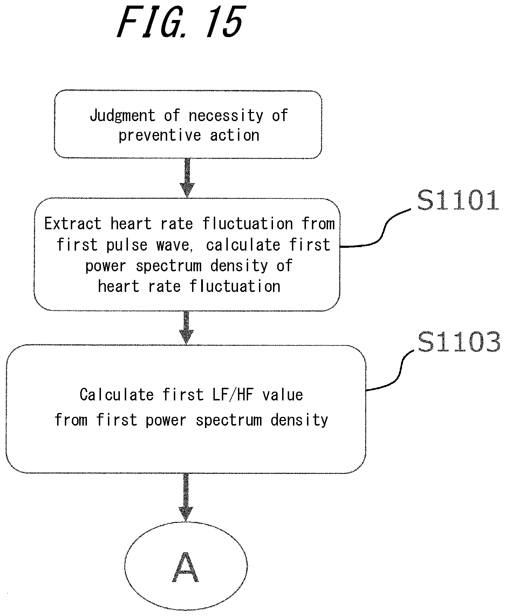

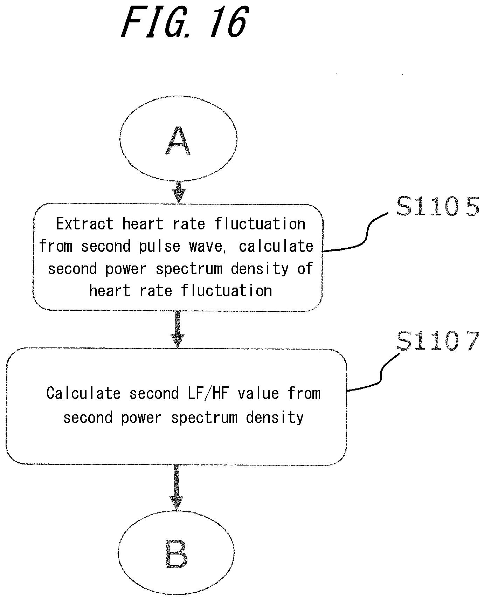

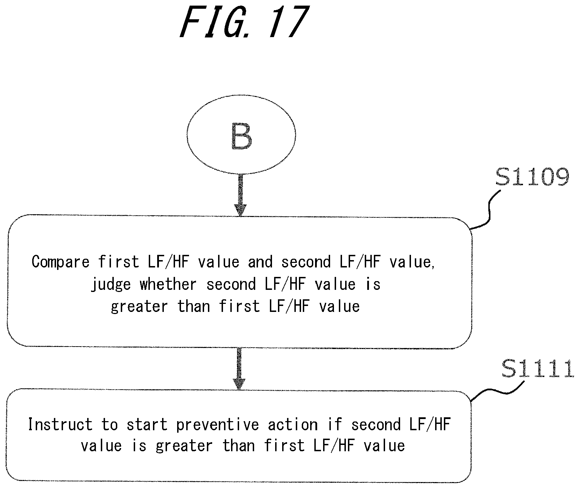

Next, the judgment of necessity of a preventive action by the smartphone 103 as indicated in step S827 of FIG. 12 is explained with reference to FIGS. 15, 16, and 17. FIGS. 15, 16, and 17 are flowcharts for the smartphone 103 to judge necessity of a preventive action as illustrated in step S827 of FIG. 12. The operations illustrated in FIGS. 15, 16, and 17 are executed by the controller 501 of the smartphone 103 working together with a program. In the flowcharts described below, a step represented by a circled letter indicates connection to a step represented by the same circled letter in another figure. For example, the circled A in FIG. 15 is connected to the circled A in FIG. 16.

Based on the atmospheric pressure information stored in the storage 507, the smartphone 103 identifies the time point T1 at which the atmospheric pressure starts to drop, as described above in step S805. The time 9:00 is T1 in the graph in FIG. 13.

As described above in step S811, the smartphone 103 measures a first pulse wave as first biological information at the identified time point T1. For example, the smartphone 103 displays a measurement start button on the touch panel display and starts the measurement operation by each measurement unit of the wearable device 101 upon detecting that the user has touched the measurement start button.

The smartphone 103 extracts the heart rate variability from the first pulse wave and calculates a power spectrum density of the heart rate variability as a first power spectrum density (S1101).

The smartphone 103 extracts the high-frequency (HF) component and the low-frequency (LF) component from the first power spectrum density. The smartphone 103 then divides the LF component by the HF component to calculate LF/HF as a first LF/HF (S1103). The LF component is a component in the region of 0.05 Hz to less than 0.15 Hz in the power spectrum density, and the HF component is a component in the region of 0.15 Hz to less than 0.40 Hz in the power spectrum density. The LF component and the HF component may be components in an appropriate frequency range other than the aforementioned values.

As described above in step S821, the smartphone 103 measures a second pulse wave as second biological information at time point T2, which occurs after a predetermined time t elapses from the identified time point T1. For example, the smartphone 103 displays a measurement start button on the touch panel display and starts the measurement operation by each measurement unit of the wearable device 101 upon detecting that the user has touched the measurement start button.

The wearable device 101 may be configured to measure biological information automatically when being worn by the user, without an instruction from the user. The wearable device 101 may, for example, use a living body detection sensor, such as an illuminance sensor, to detect wearing by the user. The wearable device 101 may, for example, start measurement of biological information based on a measurement instruction signal transmitted from the smartphone 103. When the atmospheric pressure information changes, the smartphone 103 may transmit the measurement instruction signal to the wearable device 101 even without a user instruction.

The smartphone 103 extracts the heart rate variability from the second pulse wave and calculates a power spectrum density of the heart rate variability as a second power spectrum density (S1105).

The smartphone 103 extracts the high-frequency (HF) component and the low-frequency (LF) component from the second power spectrum density. The smartphone 103 then divides the LF component by the HF component to calculate LF/HF as a second LF/HF (S1107).

The smartphone 103 compares the first LF/HF value with the second LF/HF value and judges whether the second LF/HF value is greater than the first LF/HF value (S1109). Here, the LF/HF value indicates the degree of relaxation or stress of the user. A small LF/HF value indicates that the user is relaxed and is feeling little stress, whereas a large LF/HF value indicates that the user is not relaxed and is feeling significant stress.

When the second LF/HF is judged to be larger than the first LF/HF value, the smartphone 103 provides notification of the start of a preventive action (S1111).

The smartphone 103 provides an instruction to start the preventive action when a preventive action needs to be performed (S829). In this case, the preventive action may start in response to user instruction. When the wearable device 101 is being worn by the user, the wearable device 101 may, for example, vibrate, issue an instruction for a massage, or play back predetermined music, even without an instruction from the user to start a preventive action. The wearable device 101 may, in this case, include a living body detection sensor such as an illuminance sensor for detecting wearing by the user. The wearable device 101 may similarly vibrate, issue an instruction for a massage, play back predetermined music, or the like without an instruction from the user to start a preventive action even in the case of the wearable device 101 not being worn by the user.

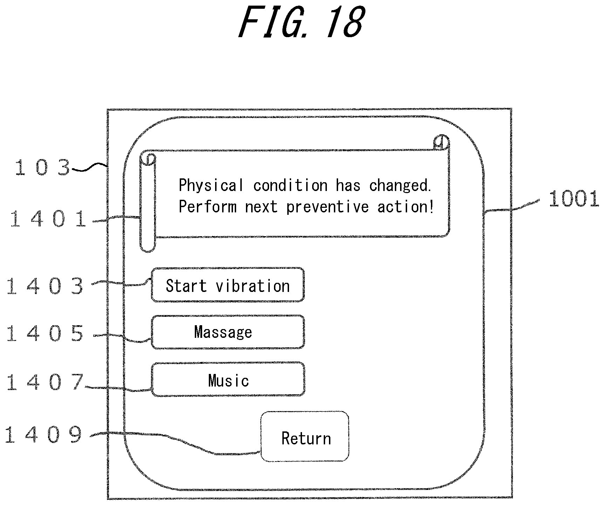

The instruction operation in step S829 is described with reference to FIG. 18. FIG. 18 is a schematic view of a screen, displayed on the touch panel display 1001 of the smartphone 103, instructing to start a preventive action.

As illustrated in FIG. 18, the touch panel display 1001 displays a message 1401 providing notification of a change in physical condition and instructing to perform a preventive action.

The touch panel display 1001 displays a vibration start button 1403, a massage button 1405, and a music start button 1407 as buttons for starting preventive actions.

When the user touches the vibration start button 1403, the smartphone 103 transmits an instruction to start vibration to the vibration unit of the wearable device 101.

When the user touches the massage button 1405, the smartphone 103 reports a massage action. This report is provided by image or audio.

When the user touches the music start button 1407, music is outputted from at least one of a speaker included in the reporting interface 511 of the smartphone 103, the vibration units 205L, 205R of the wearable device 101, and a speaker of the wearable device 101. This music may be the above-described music including sounds at Solfeggio frequencies.

The wearable device 101 performs a preventive action based on the instruction from the smartphone 103 to start a preventive action (S831). Examples of preventive actions performed by the wearable device 101 include causing the vibration units 205R, 205L to vibrate and outputting predetermined audio, such as sound at a Solfeggio frequency, from the vibration unit or speaker.

It suffices for at least one of the vibration units 205R, 205L to vibrate during the operation to cause the vibration units 205R, 205L to vibrate. The vibration operations of the vibration units 205R, 205L may differ. The vibration operation of the vibration units 205R, 205L may be vibration at a constant rhythm, vibration at a random rhythm, vibration synchronized with a song, vibration with changing intensity, or any appropriate combination of these vibrations.

The smartphone 103 performs a preventive action, such as displaying an image or providing audio notification of massage information for physical condition management (S833).

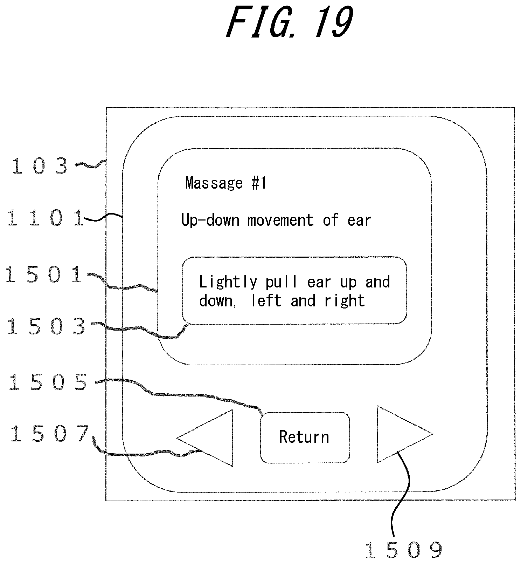

This preventive action by the smartphone 103 is described with reference to FIG. 19. FIG. 19 is a schematic view of a massage action reporting screen displayed on the touch panel display 1101 of the reporting interface 511 of the smartphone 103. In other words, the reporting interface 511 functions as a second reporting interface for reporting massage information.

As illustrated in FIG. 19, a message 1501 with the type of massage, a message 1503 with the method of performing the massage, a button 1505 to return to the previous screen, a button 1507 to return to the previous type of massage, and a button 1509 to advance to the next type of massage are displayed on the touch panel display 1101 of the smartphone 103.

The user can perform a massage himself to prevent a change in physical condition by following the message 1503 indicated in FIG. 19. In addition to an image-based instruction, the report of the massage action may be an audio-based instruction, an instruction screen indicating the address of a website carrying information on performing massages, an instruction screen introducing magazines or books carrying information on performing massages, or a television or an instruction screen introducing a radio program emitting information on performing massages. In addition to an ear exercise, the massage action of the present disclosure may be a hand exercise, a foot exercise, a back exercise, a deep breath, a walk, loosening up muscles, stretching, massaging the fingers of a hand or foot, pressing a predetermined location on the skin, pushing a pressure point, a neck exercise, an eye exercise, a mouth exercise, an exercise for another body part, or any combination of these. An explanation of the aforementioned massage actions may be displayed on the touch panel display 1101 of the smartphone 103.

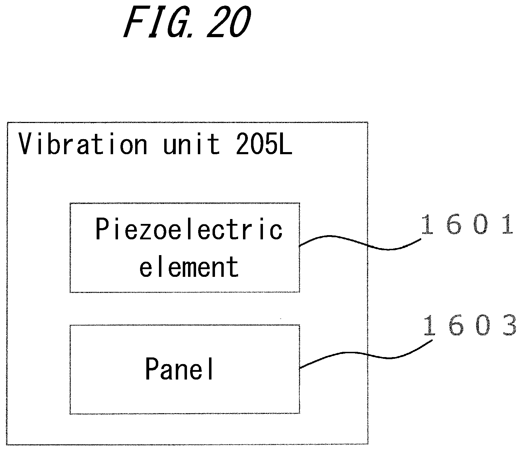

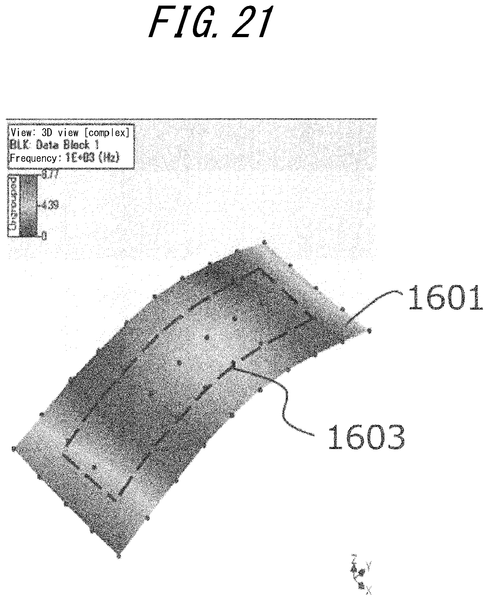

Next, the vibration units 205R, 205L of the wearable device 101 are described with reference to FIG. 20 and FIG. 21. FIG. 20 is a block diagram illustrating the configuration of the vibration unit 205L. FIG. 21 is a schematic view of operation of the vibration unit 205L. The vibration unit 205L is described below, but the same description holds for the vibration unit 205R as well.

As illustrated in FIG. 20, the vibration unit 205L includes a piezoelectric element 1601 that flexes and a panel 1603 that vibrates by being bent directly by the piezoelectric element 1601.

FIG. 21 is a schematic view illustrating flexing of the panel 1603 due to the piezoelectric element 1601. Since the panel 1603 is bent directly by the piezoelectric element 1601 and vibrates, the central area of the panel flexes greatly, swelling upward as compared to the ends.

The vibration unit 205L mainly causes the user to hear human body vibration sound through vibration. Depending on the area of the panel, air-conducted sound may also be produced. Air-conducted sound is sound that reaches the user's auditory nerve when vibrations caused by a vibrating object travel through the external ear canal to the eardrum, and the eardrum vibrates. Human body vibration sound is sound that is transmitted to the user's auditory nerve through a portion of the user's body (such as the cartilage of the outer ear) that touches a vibrating object. A component transmitted to the respiratory tract by vibration inside the external ear canal may be included in the human body vibration sound. In particular, when the area of the panel 1603 is small, it is known that vibration of the ear causes harmonics, at or above the sixth harmonic and sufficiently louder than background noise, to occur at three or more locations. The combination of these harmonic components allows sound to be heard sufficiently with a small panel 1603 (such as an elongated shape measuring 3 cm long by 1 cm wide or less). The harmonic components contribute in particular to clarity of sound and are therefore also suitable in hearing aids for presbycusis, which is characterized by difficulty hearing.

The vibration unit 205L also provides vibration to the user. A certain vibration-based massage effect is obtained at the user's skin surface, and the body inside the skin surface, where the vibration is provided by the vibration unit 205L. The vestibular nerve may be relaxed by vibration of the vibration unit 205L.

The piezoelectric element 1601 is formed by elements that, upon application of an electric signal (voltage), either expand and contract or bend (flex) in accordance with the electromechanical coupling coefficient of their constituent material. The piezoelectric element 1601 is attached to the panel 1603 directly by double-sided tape.

Ceramic or crystal elements, for example, may be used. The piezoelectric element 1601 may be a unimorph, bimorph, or laminated piezoelectric element. Examples of a laminated piezoelectric element include a laminated unimorph element with layers of unimorph (for example, approximately 16 to 100 stacked layers) and a laminated bimorph element with layers of bimorph (for example, approximately 16 to 48 stacked layers). Such a laminated piezoelectric element may be a laminated structure formed by a plurality of dielectric layers composed of, for example, lead zirconate titanate (PZT) and electrode layers disposed between the dielectric layers. Unimorph expands and contracts upon the application of an electric signal (voltage), and bimorph bends upon the application of an electric signal (voltage).

The panel 1603 is, for example, made from a hard material such as glass or sapphire, or a synthetic resin such as acrylic or polycarbonate. An exemplary shape of the panel 1603 is a plate, and the shape of the panel 1603 is described below as being a plate. For example, the plate is approximately 2 cm to 5 cm long and approximately 0.5 cm to 2 cm wide. The piezoelectric element 1601 is attached directly to the panel 1603 by double-sided tape or the like.

Vibration of the vibration units 205R, 205L may at least include vibration of approximately 5 Hz to 10 Hz or less, centered on 7 Hz, to stimulate the vestibular nerve and the region near the inner ear. When this vibration stimulates the vestibular nerve and the region near the inner ear and makes the parasympathetic nerves of the user active, the user relaxes, and the user's physical condition might improve. Vibration of the vibration units 205R, 205L may be outside of the aforementioned frequency range.

As described above, the vibration units 205R, 205L output audio while vibrating. In other words, the vibration units 205R, 205L function as an output interface that outputs audio. There may be one or more types of music as information of the music outputted by the vibration units 205R, 205L. This music may be music that allows the user to relax. This music may, for example, be music at a Solfeggio frequency. The Solfeggio frequencies are, for example, 174 hz, 285 hz, 396 hz, 417 hz, 528 hz, 639 hz, 741 hz, 852 hz, and 963 hz.

In the present disclosure, the smartphone 103 detects a change in atmospheric pressure. A plethysmogram, which is biological information of the user, is detected when the atmospheric pressure drops. The plethysmogram, which is biological information of the user, is detected again in the present disclosure after a predetermined length of time from when the atmospheric pressure drops.

In the present disclosure, values related to LF/HF are calculated from two plethysmograms and are compared. A preventive action is reported to the user based on the comparison of these two values related to LF/HF in the present disclosure. Therefore, the user's physical condition can be managed appropriately in the present disclosure based on the user's biological information and atmospheric pressure information.

Second Embodiment Using Blood Flow Meter

Next, a second embodiment of an electronic device is described. The electronic device of the second embodiment has the configuration of the electronic device of the first embodiment, with the plethysmograph in the measurement unit of the wearable device 101 being replaced by a blood flow meter. The second embodiment of the present disclosure is described below, focusing on the main differences from the first embodiment.

In the electronic device of the second embodiment, the measurement unit 201L of the wearable device 101 illustrated in FIG. 5 is replaced by a measurement unit 1801L, and the measurement unit 201R is replaced by a measurement unit 1801R. The electronic device of the second embodiment may have the configuration of the wearable device 101 illustrated in FIG. 5 with the addition of the measurement units 1801L, 1801R.

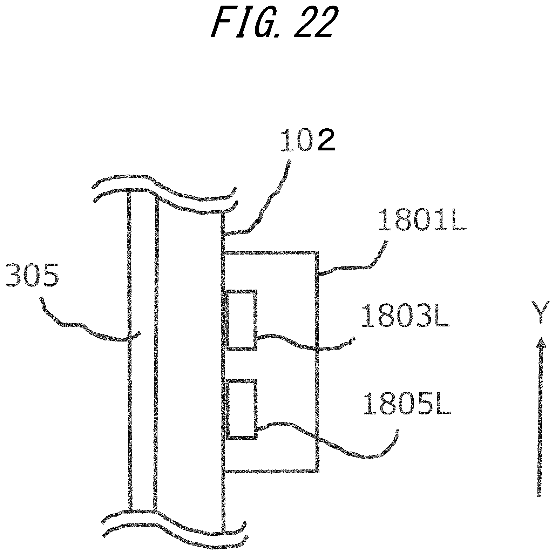

FIG. 22 is a schematic configuration diagram of the measurement unit 1801L of an electronic device according to the second embodiment. The configuration of the measurement unit 1801R is similar to the configuration illustrated in FIG. 22.

The measurement unit 1801L includes an optical emitter 1803L and an optical detector 1805L. The measurement unit 1801L irradiates a measurement beam from the optical emitter 1803L onto the superficial temporal artery 305, which is a measured part. The measurement unit 1801L uses the optical detector 1805L to acquire reflected light (scattered light), from the superficial temporal artery 305, corresponding to the irradiated measurement beam. The measurement unit 1801L transmits a photoelectric conversion signal of the scattered light acquired by the optical detector 1805L to the controller 501.

The optical emitter 1803L emits laser light in response to control by the controller 501. For example, the optical emitter 1803L irradiates the measured part with laser light, as a measurement beam, that has a wavelength capable of detecting a predetermined component included in blood. The optical emitter 1803L is, for example, configured by one laser diode (LD).

The optical detector 1805L detects scattered light of the measurement beam from the measured part. The optical detector 1805L may, for example, be configured by a photodiode (PD).

The controller 501 calculates the blood flow at the measured part as information related to blood flow based on the photoelectric conversion signal received from the measurement unit 1801L. A technique for the controller 501 to measure the amount of blood flow using the Doppler shift is now described.

The controller 501 may transmit the photoelectric conversion signal received from the measurement unit 1801L to the server 109, and the controller 521 of the server 109 may calculate the blood flow at the measured part as the information related to blood flow.

In the body tissue, scattered light that is scattered from moving blood cells undergoes a frequency shift (Doppler shift), due to the Doppler effect, proportional to the speed of travel of the blood cells within the blood. The controller 501 detects the beat signal produced by interference between scattered light from still tissue and the scattered light from moving blood cells.

This beat signal represents strength as a function of time. The controller 501 then turns the beat signal into a power spectrum that represents power as a function of frequency. In the power spectrum of this beat signal, the Doppler shift frequency is proportional to the speed of blood cells. Also, the power corresponds to the amount of blood cells in the power spectrum of this beat signal. The controller 501 calculates the blood flow by multiplying the power spectrum of the beat signal by the frequency and integrating.

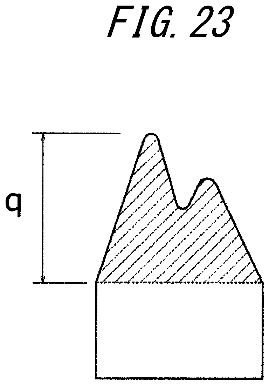

FIG. 23 is a schematic view illustrating an example blood flow waveform acquired by the measurement unit 1801L. The blood flow waveform illustrated in FIG. 23 is the blood flow waveform of one pulse beat of a subject. The blood flow waveform illustrated in FIG. 23 indicates a change in the blood flow and is generated by the controller 501 based on the calculated blood flow, for example. In FIG. 23, the hatched portion q is the portion of the blood flow that varies together with the beat.

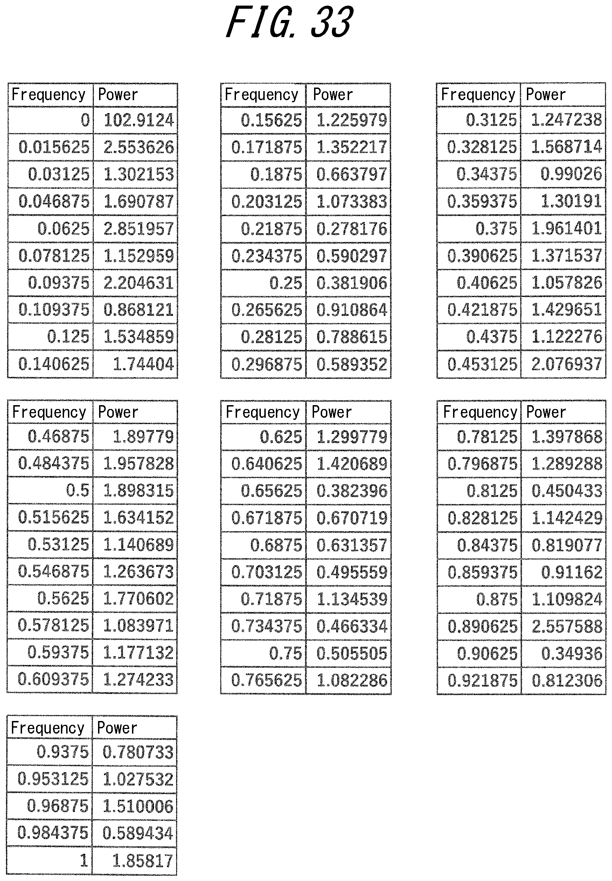

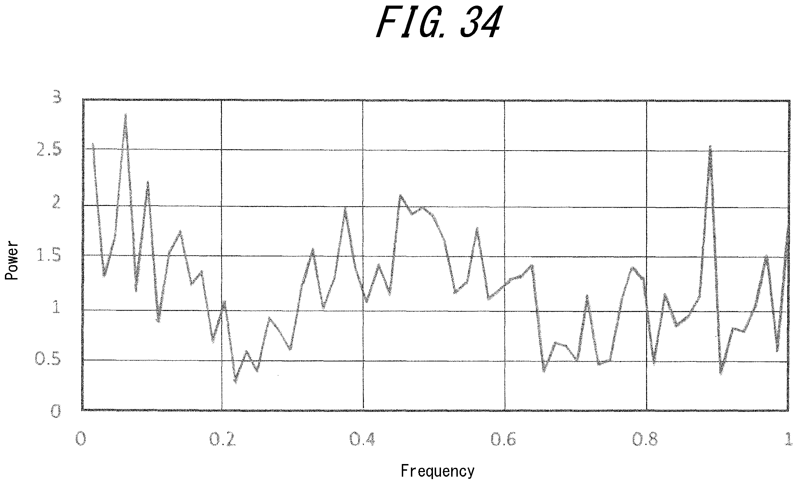

Here, the relationship between frequency and power spectrum for the blood flow measured by a blood flow meter is described with reference to FIG. 33 and FIG. 34. FIG. 33 is a table illustrating the relationship between frequency and power for the blood flow obtained with a blood flow meter, and FIG. 34 is a graph of the table in FIG. 33.

As illustrated in FIG. 33 and FIG. 34, a power spectrum analysis of blood flow by frequency yields a power distribution at each frequency.

In the second embodiment, the pulse is calculated based on the blood flow measured by the measurement unit. This calculated pulse is then used to calculate and compare values related to LF/HF at each of time point T1 and time point T2, like the above-described first embodiment. A preventive action is reported to the user based on the comparison of these two values related to LF/HF in the present disclosure. Therefore, the user's physical condition can be managed appropriately in the present disclosure based on the user's biological information and atmospheric pressure information.

Third Embodiment Using an Atmospheric Pressure Sensor

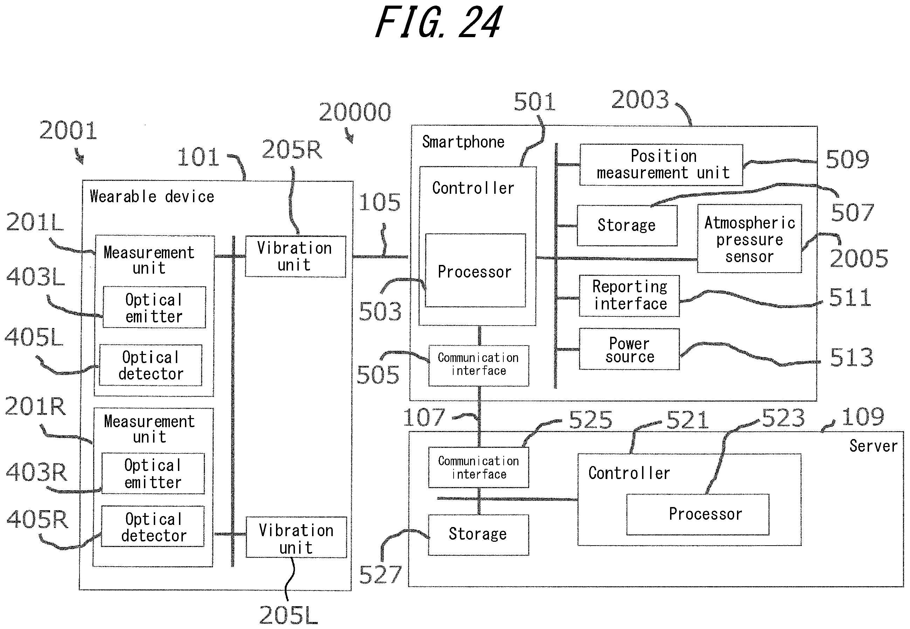

Next, a third embodiment of an electronic device according to the present disclosure is described with reference to FIG. 24. FIG. 24 is an internal block diagram of a physical condition management system 20000 that uses a wearable system 2001, which is an electronic device of the third embodiment. In the third embodiment, a smartphone 2003 includes an atmospheric pressure sensor 2005. In FIG. 24, the same reference numerals are used for functions and operational members that are the same as in FIG. 5. The third embodiment of the present disclosure is described below, focusing on the main differences from the first embodiment.

In the third embodiment, the smartphone 2003 includes the atmospheric pressure sensor 2005, as illustrated in FIG. 24.

The atmospheric pressure sensor 2005 detects the atmospheric pressure at the location of the smartphone 2003. The atmospheric pressure detected by the atmospheric pressure sensor 2005 is stored in the storage 507. The atmospheric pressure sensor 2005 may be a sensor that measures the atmospheric pressure by detecting a change in capacitance caused by a change in atmospheric pressure, a sensor that measures the atmospheric pressure by measuring deformation, due to external pressure, as a change in electrical resistance yielded by the piezoresistive effect, or the like. Any other sensor can be used as the atmospheric pressure sensor 2005 of the present disclosure.

The controller 501 controls operations of the atmospheric pressure sensor 2005. For example, the controller 501 detects the atmospheric pressure with the atmospheric pressure sensor 2005 every hour. The atmospheric pressure detected by the controller 501 becomes the information related to atmospheric pressure used in the above-described first embodiment. The controller 501 may detect the atmospheric pressure with the atmospheric pressure sensor 2005 at a time interval other than every hour. For example, the controller 501 may detect the atmospheric pressure with the atmospheric pressure sensor 2005 at any time interval, such as every five minutes, every 30 minutes, every two hours, every six hours, or every day.

In this way, the smartphone 2003 in the third embodiment can detect information related to atmospheric pressure using the atmospheric pressure sensor 2005 and can create atmospheric pressure information. The third embodiment enables appropriate physical condition management even when atmospheric pressure information cannot be acquired from an external source.

Fourth Embodiment Using an Atmospheric Pressure Threshold

Next, a fourth embodiment of an electronic device is described. The configuration of the fourth embodiment is similar to that of the first embodiment described with reference to FIG. 5 and the like. In the fourth embodiment, the timing of measurement of biological information differs from the first embodiment. The fourth embodiment of the present disclosure is described below, focusing on the main differences from the first embodiment.

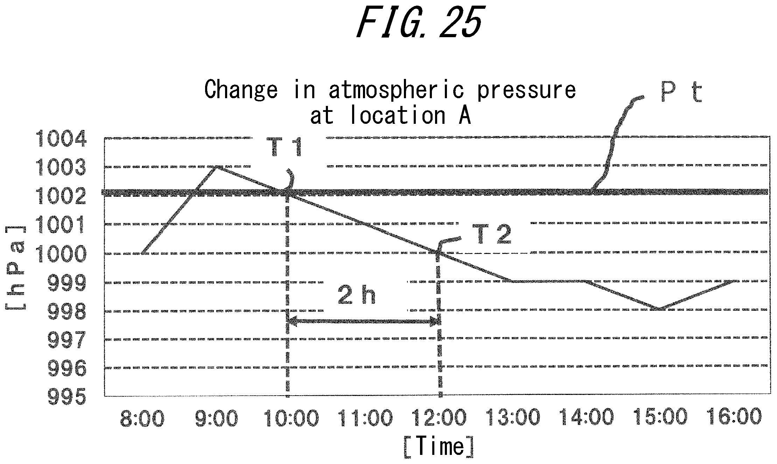

FIG. 25 is a graph illustrating atmospheric pressure analysis operations in an electronic device of the fourth embodiment. The controller 501 of the smartphone 103 of the fourth embodiment judges whether the atmospheric pressure has become equal to or less than a predetermined threshold in the atmospheric pressure analysis operation (S805) of the flowchart illustrated in FIG. 12.

In the example in FIG. 25, a predetermined threshold Pt is 1002 [hPa]. The controller 501 takes the time point at which the atmospheric pressure becomes equal to or less than the predetermined threshold Pt as a timing T1 for measuring biological information. The controller 501 stores the biological information measured at time point T1 in the storage 507. This predetermined threshold Pt may be any value other than 1002 [hPa], and a plurality of thresholds may be used.

Operations by the electronic device of the fourth embodiment after measuring the biological information at timing T1 are similar to the above-described first embodiment.

In this way, the electronic device of the fourth embodiment can measure the biological information at the point in time at which the atmospheric pressure becomes equal to or less than the predetermined threshold Pt, allowing more appropriate physical condition management that matches the user's physical condition.

Fifth Embodiment with Biological Sensor Mounted in Smartphone

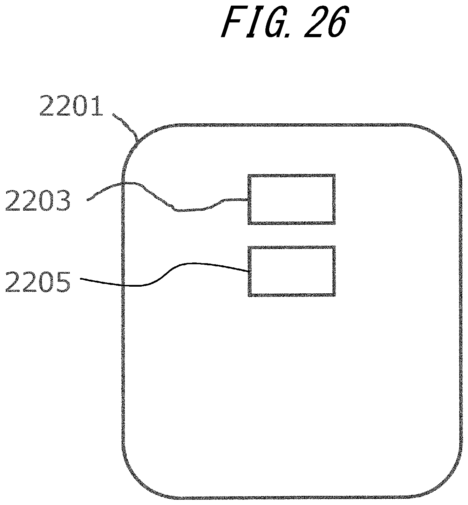

Next, an electronic device of a fifth embodiment of the present disclosure is described. The fifth embodiment differs from the first embodiment in that the smartphone forming part of the wearable system as an electronic device includes a measurement unit that measures biological information of a user. The remaining configuration is similar between the fifth embodiment and the first embodiment. In other words, the smartphone 103 illustrated in FIG. 5 is replaced in the fifth embodiment by the smartphone 2201 described below. The fifth embodiment of the present disclosure is described below, focusing on the main differences from the first embodiment. FIG. 26 is a schematic view of the smartphone 2201 used in an electronic device of the fifth embodiment.

In the above-described embodiment, the measurement unit that measures the biological information of the user is in the wearable device 101, but the present disclosure is not limited to this case. A measurement unit that measures the biological information of the user may be in the smartphone 103. Instead of the measurement unit 201L or the measurement unit 201R illustrated in FIG. 5, an LED light of the smartphone 103 may be used as an optical emitter, and a camera of the smartphone 103 may be used as an optical detector. A biological sensor may be provided in the smartphone 103 as an electronic device of the fifth embodiment of the present disclosure.

As illustrated in FIG. 26, the smartphone 2201 includes an optical detector 2203 that detects light and an optical emitter 2205 that emits light. The optical detector 2203 is, for example, a camera. The optical emitter 2205 is, for example, an LED. The optical detector 2203 and the optical emitter 2205 form a plethysmograph.

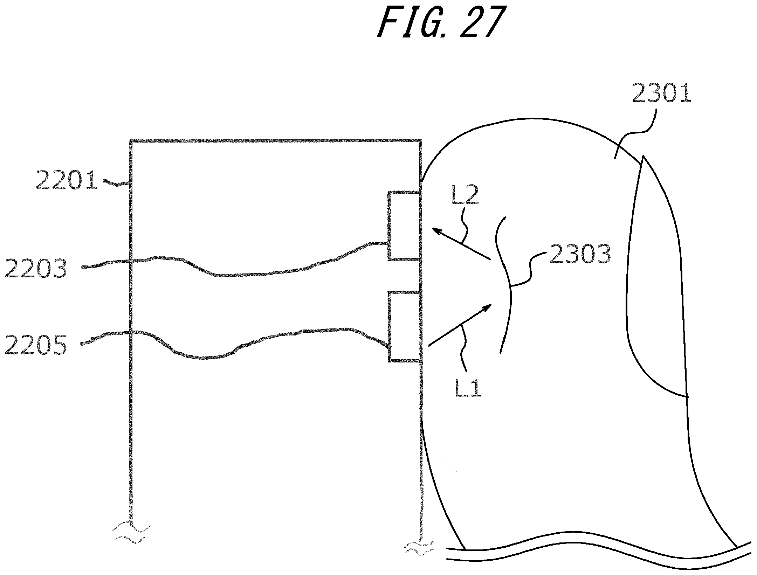

FIG. 27 is a side schematic view of the smartphone illustrated in FIG. 26. When the smartphone 2201 measures the biological information of the user, the user's finger 2301 covers at least a portion of the optical emitter 2203 and the optical detector 2205, as illustrated in FIG. 27. The user's finger may cover at least a portion of the optical emitter 2203 and the optical detector 2205 at different points in time.

Light L1 emitted from the optical emitter 2205 becomes scattered light L2 scattered at a blood vessel 2303 of the user's finger 2301 and is incident on the optical detector 2203.

In this way, the optical detector 2203 and the optical emitter 2205 form a plethysmograph in the smartphone 2201. The optical detector 2203 and the optical emitter 2205 may configure a blood flow meter.

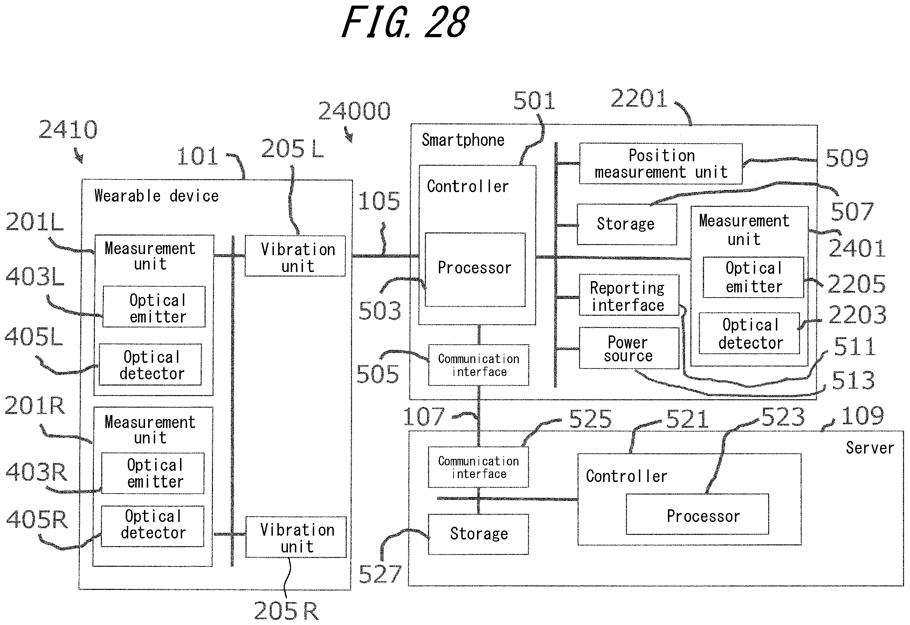

A physical condition management system 24000 using the smartphone 2201 illustrated in FIG. 26 is described with reference to FIG. 28. FIG. 28 is an internal block diagram of the physical condition management system 24000 using the smartphone 2201 illustrated in FIG. 26. In FIG. 26, the same reference numerals are used for members performing the same operations as the members in FIG. 5. The fifth embodiment of the present disclosure is described below, focusing on the main differences from the first embodiment.

As illustrated in FIG. 28, the physical condition management system 24000 includes a wearable system 2410, which is the fifth embodiment of an electronic device according to the present disclosure, and the server 109. The wearable system 2410 includes the wearable device 101 and the smartphone 2201.

The smartphone 2201 includes the measurement unit 2401. The measurement unit 2401 includes the optical emitter 2203 and the optical detector 2205. Operations by the measurement unit 2401 are controlled by the controller 501. In the example in FIG. 28, the wearable device 101 includes the measurement units 201L, 201R. In the fifth embodiment, however, at least one of the measurement units 201L, 201R may be omitted.

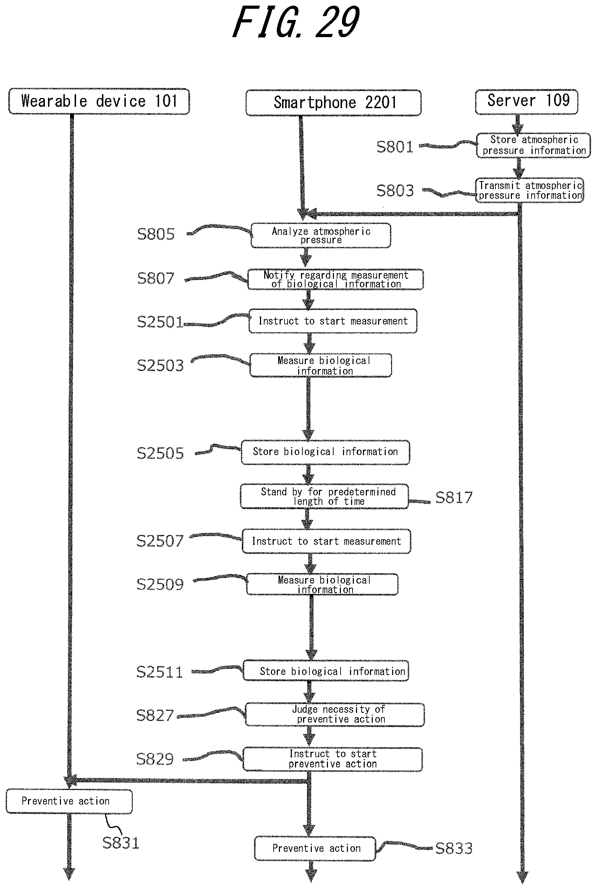

Next, with reference to FIG. 29, the operations of the physical condition management system 24000 illustrated in FIG. 28 are described. FIG. 29 is a flowchart of operations of the physical condition management system 24000 illustrated in FIG. 28. In FIG. 29, the same step numbers are used for operations that are similar to those of the flowchart in FIG. 12.

As illustrated in FIG. 29, the server 109 first stores atmospheric pressure information in the storage 527 (S801). This atmospheric pressure information is, for example, atmospheric pressure information distributed by the government or the like. The atmospheric pressure information may also be atmospheric pressure information created by a private institution. The server 109 transmits the atmospheric pressure information to the smartphone 2201 (S803).

After receiving the atmospheric pressure information from the server 109, the smartphone 2201 analyzes the atmospheric pressure information (S805). The atmospheric pressure analysis operation in step S805 is similar to the case described above with reference to FIG. 12.

Based on the analysis result of the atmospheric pressure information, the smartphone 2201 then provides notification of the start of biological information measurement (S807). Notification of the start of measurement is, for example, provided by the display of an image on a touch panel display, or by audio or vibration.

Based on the analysis result of the atmospheric pressure, the smartphone 2201 issues an instruction to start measurement of biological information to the measurement unit 2401 (S2501). Measurement of biological information may instead begin in response to the user touching an instruction button, displayed on the touch panel display, for starting biological information measurement. The smartphone 2201 may also be configured so that measurement of the biological information starts in response to user instruction or without user instruction.

After receiving the instruction to start measurement of biological information from the smartphone 2201, the measurement unit 2401 starts measurement of biological information (S2503). The measurement unit 2401 measures information indicating a plethysmogram of the user in the present disclosure. The smartphone 2201 may also issue an instruction to start measurement to the measurement units 201R, 201L of the wearable device 101.

The smartphone 2201 stores the biological information measured by the measurement unit 2401 in the storage 507 (S2505).

After storing the measurement information, the smartphone 2201 stands by for a predetermined length of time (S817). The smartphone 103 stands by for two hours in step S817. While this predetermined elapsed time is two hours in this example, the predetermined elapsed time may be any length of time other than two hours, such as five minutes, 30 minutes, one hour, six hours, one day, or the like.

After standing by for this predetermined length of time, the smartphone 2201 instructs the measurement unit 2401 to start measuring biological information (S2507). The smartphone 2201 may issue an instruction to start measurement to the measurement units 201R, 201L of the wearable device 101.

After receiving the instruction to start measurement of biological information from the smartphone 2201, the measurement unit 2401 starts measurement of biological information (S2509). The measurement unit 2401 measures information indicating a plethysmogram of the user in the present disclosure. The smartphone 2201 may also issue an instruction to start measurement to the measurement units 201R, 201L of the wearable device 101.

The smartphone 2201 stores the biological information measured by the measurement unit 2401 in the storage 507 (S2511).

The smartphone 2201 compares two pieces of information related to biological information stored in the storage 507. When a predetermined condition is satisfied, the smartphone 2201 judges whether a preventive action is to be performed (S827). When judging that a preventive action is to be performed, the smartphone 2201 may, for example, instruct the user to start a preventive action as in the above-described step S829.

The wearable device 101 performs a preventive action based on the instruction from the smartphone 2201 to start a preventive action (S831). Examples of preventive actions performed by the wearable device 101 include causing the vibration unit to vibrate and outputting predetermined audio from the vibration unit or speaker. The wearable device 101 may perform a preventive action either in response to user instruction or, in the absence of user instruction, in response to the instruction from the smartphone 2201 to start a preventive action.

The smartphone 2201 performs a preventive action, such as displaying an image or providing audio notification of massage information for physical condition management (S833).

In this way, the fifth embodiment of the present disclosure achieves similar effects to the first embodiment and also allows measurement of biological information with the smartphone 2201, thereby improving convenience for the user.

The vibration units 205L, 205R illustrated in FIG. 28 may be provided in the smartphone 2201 of the fifth embodiment. In this case, the measurement unit 2401 that measures the biological information of the user and one of the vibration units 205L, 205R are provided in the smartphone 2201. An apparatus that outputs sound, such as a dynamic speaker or a piezoelectric element speaker, may further be provided in the smartphone 2201 in this case. This configuration allows the smartphone 2201 alone to measure biological information of the user, massage the user by vibration, and play back music. The wearable device 101 can therefore be omitted in this case. In other words, only the smartphone 2201 is the electronic device in this case.

Earphone-Type Sixth Embodiment

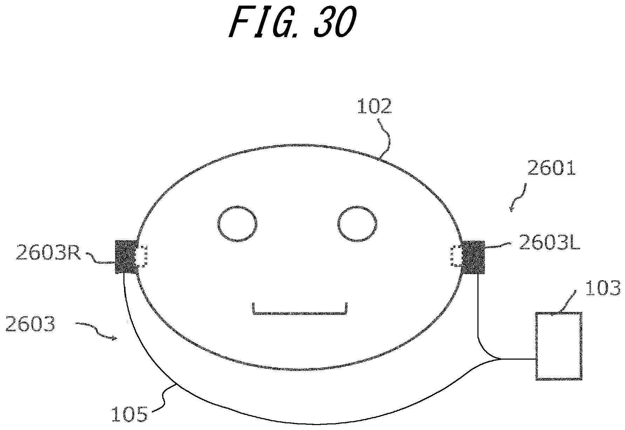

Next, a sixth embodiment of the present disclosure is described below with reference to FIG. 30. The electronic device of the sixth embodiment differs from the wearable system 100 illustrated in FIG. 1 in that the wearable device 101 becomes an earphone-type wearable device 2603. The sixth embodiment is otherwise similar to the first embodiment. The sixth embodiment of the present disclosure is described below, focusing on the main differences from the first embodiment.

FIG. 30 is a schematic configuration diagram of the wearable system 2601, which is an electronic device of the sixth embodiment. As illustrated in FIG. 30, the wearable system 2601 includes a smartphone 103 and a wearable device 2603 connected to the smartphone 103 by a cable 105. The internal configuration of the smartphone 103 is similar to the configuration illustrated in FIG. 5.

The wearable device 2603 includes a right-ear earphone 2603R and a left-ear earphone 2603L. The right-ear earphone 2603R and the left-ear earphone 2603L are connected to each other by the cable 105. The right-ear earphone 2603R and the left-ear earphone 2603L may be connected to the smartphone 103 by wireless communication instead of by the cable 105.

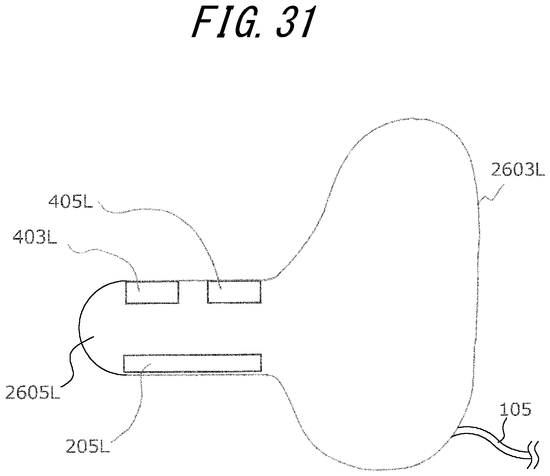

FIG. 31 is a schematic configuration diagram of the left-ear earphone 2603L illustrated in FIG. 30. The schematic configuration of the right-ear earphone 2603R illustrated in FIG. 30 is similar to FIG. 31. The operations of the right-ear earphone 2603R illustrated in FIG. 30 are similar to the operations of the left-ear earphone 2603L described below.

As illustrated in FIG. 31, the left-ear earphone 2603L includes a left-ear outer-ear insertion portion 2605L. An optical emitter 403L and an optical detector 405L are included in the outer-ear insertion portion 2605L at the portion in contact with the external ear canal. The outer-ear insertion portion 2605L includes a vibration unit 205L.

The optical emitter 403L and the optical detector 405L of the outer-ear insertion portion 2605L can measure the biological information of the user. The vibration unit 205L of the outer-ear insertion portion 2605L can generate audio and can also perform a massage by applying vibration. The optical emitter 403L and the optical detector 405L of the outer-ear insertion portion 2605L form a plethysmograph, like the above-described first embodiment. The optical emitter 403L and the optical detector 405L of the outer-ear insertion portion 2605L may form a blood flow meter, like the above-described second embodiment.

The configuration illustrated in FIG. 31 allows the user to wear the left-ear earphone 2603L in the left outer ear. The same effects as in the first embodiment can also be achieved.

Seventh Embodiment for Providing Notification of Physical Condition After Preventive Action

Next, an electronic device of a seventh embodiment is described. An electronic device of the seventh embodiment has the configuration of the above-described electronic device of the first embodiment while also providing notification of the physical condition of the user after a preventive action. The seventh embodiment of the present disclosure is described below, focusing on the main differences from the first embodiment.

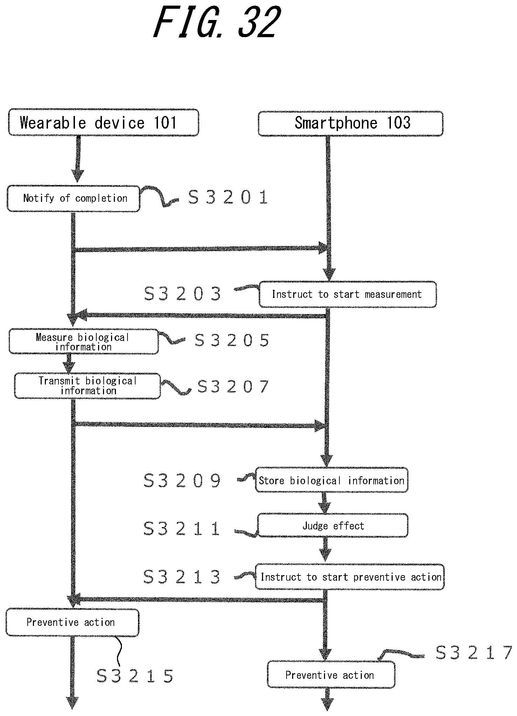

FIG. 32 is a flowchart of operations by the seventh embodiment of an electronic device of the present disclosure. As illustrated in FIG. 32, the operations of the seventh embodiment differ from the operations of the first embodiment in that the operations illustrated in FIG. 32 are added after the preventive action S831 and the preventive action S833 of the first embodiment illustrated in FIG. 12.

After the preventive action S831 illustrated in FIG. 12, the wearable device 101 transmits a notification of completion of the preventive action to the smartphone 103 (S3201). This notification of completion indicates that the preventive action described in S831 of FIG. 12 is complete. When a preventive action is not performed in the wearable device 101, transmission of this notification may be omitted.

The smartphone 103 instructs the wearable device 101 to start measuring biological information (S3203). After receiving the instruction to measure biological information, the wearable device 101 measures biological information (S3205). Here, the wearable device 101 measures a plethysmogram. The wearable device 101 then transmits the measured biological information to the smartphone 103 (S3207). The smartphone 103 may instruct the wearable device 101 to start measuring biological information either in response to user instruction or without user instruction. The timing at which the smartphone 103 instructs the wearable device 101 to start measuring biological information in step S3203 may, for example, be immediately after the smartphone 103 receives the notification of completion from the wearable device 101 in step S3201, a predetermined length of time (such as five minutes) after the instruction to start the preventive action is provided (S829), a predetermined length of time (such as five minutes) after the preventive action in step S833 is performed, or the like. The predetermined length of time may be any time other than five minutes, such as 30 seconds, one minute, one hour, or the like.

The smartphone 103 stores the biological information received from the wearable device 101 in the storage 507 (S3209). The smartphone 103 designates the stored biological information as fourth biological information.

The smartphone 103 judges the effect of the preventive action (S3211). In other words, the smartphone 103 compares the second LF/HF value at time point T2 and a fourth LF/HF value extracted from the fourth biological information.

The smartphone 103 then judges whether the value of the fourth LF/HF is greater than the value of the second LF/HF. The smartphone 103 issues an instruction to start a preventive action when the value of the fourth LF/HF is greater than the value of the second LF/HF (S3213). The issuing of an instruction to start a preventive action in step S3213 is the same as the issuing of an instruction to start a preventive action in step S829 of FIG. 12. The smartphone 103 may terminate operations when the value of the fourth LF/HF is equal to or less than the value of the second LF/HF.

In the present disclosure, the fourth LF/HF value is compared with the second LF/HF, but this configuration is not limiting. Any LF/HF value may be used as a threshold for comparison with the fourth LF/HF. The prevention action may be performed again when the fourth LF/HF is greater than this threshold, and the action may be ended when the fourth LF/HF is equal to or less than this threshold. Alternatively, the smartphone 103 may measure the body temperature and blood flow of the user and compare the values of the body temperature and blood flow at a second time point with the values of the body temperature and blood flow at a fourth time point after a massage is performed to determine whether to perform the preventive action again.

After receiving the instruction to start a preventive action, the wearable device 101 performs a preventive action (S3215). The preventive action in step S3215 is the same as the operation to start a preventive action in step S831 of FIG. 12. The smartphone 103 performs a preventive action (S3217). The preventive action in step S3217 is the same as the operation to start a preventive action in step S833 of FIG. 12.

The wearable device 101 and the smartphone 103 subsequently complete operations. The wearable device 101 may return to the operation in step S3201 after the operation in step S3215, and the smartphone 103 may return to the operation in step S3203 after the operation in step S3217.

In this way, the seventh embodiment of the present disclosure allows the smartphone 103 to perform a preventive action after judging the effect of a massage and thus allows better physical condition management.

Eighth Embodiment with CPU and Storage Mounted in Wearable Device

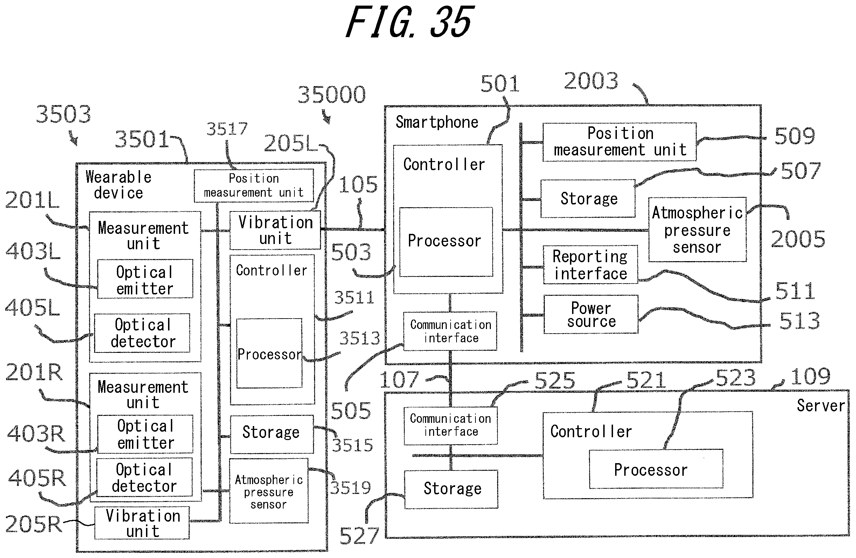

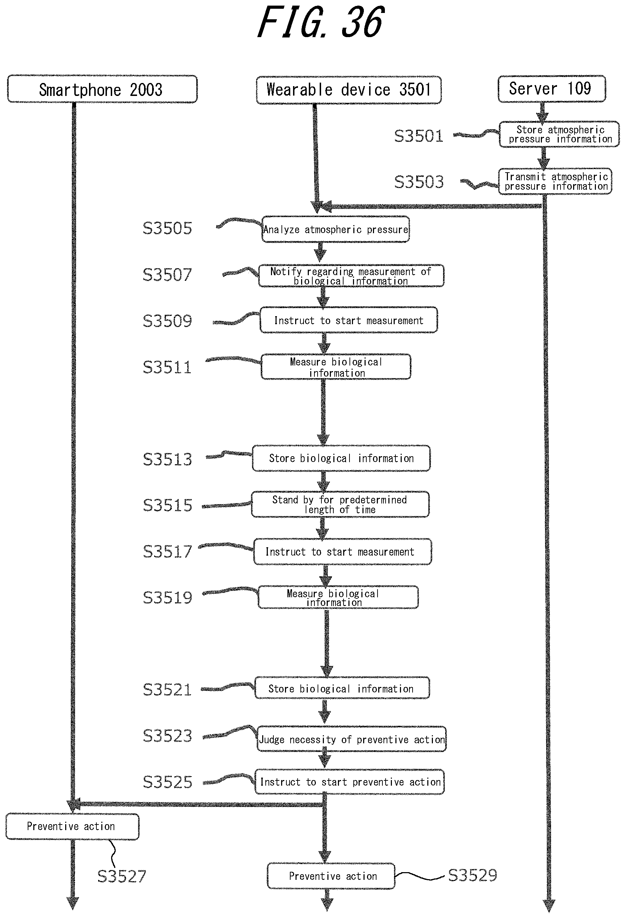

Next, an electronic device of an eighth embodiment is described with reference to FIG. 35 and FIG. 36. FIG. 35 is a block diagram of a physical condition management system 35000 that uses an electronic device of the eighth embodiment. FIG. 36 is a flowchart of operations of the physical condition management measurement system 35000 illustrated in FIG. 35.

As illustrated in FIG. 35, the electronic device of the eighth embodiment has the configuration of the above-described electronic device of the third embodiment, with the wearable device 101 being replaced by a wearable device 3501. The eighth embodiment of the present disclosure is described below, focusing on the main differences from the third embodiment.

The physical condition management system 35000 includes a wearable system 3503 and a server 109. The wearable system 3503 includes a wearable device 3501 and a smartphone 2003. The configuration and operations of the smartphone 2003 and the server 109 are similar to the configuration and operations in the above-described third embodiment.

In addition to the configuration of the wearable device 101 illustrated in FIG. 24, the wearable device 3501 includes a controller 3511, a storage 3515, a position measurement unit 3517, and an atmospheric pressure sensor 3519.

The controller 3511 is a processor that controls and manages the entire wearable system 3503, including the functional blocks of the wearable device 3501. The controller 3511 is a processor, such as a central processing unit (CPU), that executes programs prescribing control procedures. Such programs may, for example, be stored in the storage 3515 or on an external storage medium or the like connected to the wearable device 3501.

To provide control and processing capability for executing various functions, as described below in greater detail, the wearable device 3501 includes at least one processor 3513.

In various embodiments, the one or more processors 3513 may be implemented as a single integrated circuit (IC) or as a plurality of communicatively connected integrated circuits and/or discrete circuits. The one or more processors 3513 can be implemented with a variety of known techniques.

In an embodiment, the processor 3513 includes one or more circuits or units configured to execute one or more data calculation procedures or processes by executing instructions stored in related memory, for example. In another embodiment, the processor 3513 may be firmware (such as discrete logic components) configured to execute one or more data calculation procedures or processes.

In various embodiments, the processor 3513 may include one or more processors, controllers, microprocessors, microcontrollers, application specific integrated circuits (ASIC), digital signal processors, programmable logic devices, field programmable gate arrays, any combination of these devices or structures, or a combination of other known devices and structures, to execute the below-described functions of the controller 3511.

Based on atmospheric pressure information stored in the storage 3515, for example, the controller 3511 causes the reporting interface 511 to report the start of biological information measurement. Based on the instruction from the controller 3511, the reporting interface 511 uses audio, an image, vibration, or a combination of these to report the start of measurement of biological information. In other words, the reporting interface 511 has the function of a first reporting interface.

The storage 3515 can be configured by a semiconductor memory, a magnetic memory, or the like. The storage 3515 stores various information, programs for operating the smartphone 2003 and the wearable device 101, and the like. The storage 3515 may also function as a working memory. For example, the storage 3515 may store received information related to the atmospheric pressure.

Here, the atmospheric pressure information stored in the storage 3515 is similar to the atmospheric pressure information described with reference to FIG. 6 and FIG. 7.

The position measurement unit 3517 has a similar configuration to that of the position measurement unit 509 and performs similar operations. In other words, the position measurement unit 3517 measures the current position of the wearable device 3501. This position measurement may be made using a global positioning system (GPS), for example. The position information measured by the position measurement unit 3517 is stored in the storage 3515.

The atmospheric pressure sensor 3519 has a similar configuration to that of the atmospheric pressure sensor 2005 and performs similar operations. In other words, the atmospheric pressure sensor 3519 detects the atmospheric pressure at the location of the wearable device 3501. The atmospheric pressure detected by the atmospheric pressure sensor 3519 is stored in the storage 3515. The atmospheric pressure sensor 3519 may be a sensor that measures the atmospheric pressure by detecting a change in capacitance caused by a change in atmospheric pressure; a sensor that measures the atmospheric pressure by measuring deformation, due to external pressure, as a change in electrical resistance yielded by the piezoresistive effect; or the like. Any other sensor can be used as the atmospheric pressure sensor of the present disclosure.

The controller 3511 controls operations of the atmospheric pressure sensor 3519. For example, the controller 3511 detects the atmospheric pressure with the atmospheric pressure sensor 3519 every hour. The atmospheric pressure detected by the controller 3511 becomes the information related to atmospheric pressure used in the above-described first embodiment. The controller 3511 may detect the atmospheric pressure with the atmospheric pressure sensor 3519 at a time interval other than every hour. For example, the controller 3511 may detect the atmospheric pressure with the atmospheric pressure sensor 3519 at any time interval, such as every five minutes, every 30 minutes, every two hours, every six hours, or every day.

Next, with reference to FIG. 36, the operations of the physical condition management system 35000 illustrated in FIG. 35 are described. FIG. 36 is a flowchart of operations of the physical condition management system 35000 illustrated in FIG. 35.

As illustrated in FIG. 36, the server 109 first stores atmospheric pressure information in the storage 527 (S3501). This atmospheric pressure information is, for example, atmospheric pressure information distributed by the government or the like. The atmospheric pressure information may also be atmospheric pressure information created by a private institution. The atmospheric pressure information may be atmospheric pressure information inputted to the server 109 by the user. The server 109 transmits the atmospheric pressure information to the wearable device 3501 (S3503). The server 109 may transmit the atmospheric pressure information first to the smartphone 2003, and the smartphone 2003 may then transmit the atmospheric pressure information to the wearable device 3501. In other words, the atmospheric pressure information may be transmitted from the server 109 to the wearable device 3501 via the smartphone 2003. The wearable device 3501 may also acquire the atmospheric pressure information using the atmospheric pressure sensor 3519. The smartphone 2003 may measure the atmospheric pressure using the atmospheric pressure sensor 2005 and transmit the measured atmospheric pressure information to the wearable device 3501.

After receiving the atmospheric pressure information from the server 109, the wearable device 3501 analyzes the atmospheric pressure information (S3505). The atmospheric pressure analysis operation in step S3505 is similar to the case described above with reference to FIG. 12. In other words, the controller 3511 of the wearable device 3501 works together with a program stored in the storage 3515 to analyze the atmospheric pressure information.

Based on the analysis result of the atmospheric pressure information, the wearable device 3501 then provides notification of the start of biological information measurement (S3507). This notification of the start of measurement may, for example, be the output of sound from the vibration units 205L, 205R, vibration by the vibration units 205L, 205R in a predetermined pattern, or the like. Based on the analysis result of the atmospheric pressure information, the wearable device 3501 may notify the smartphone 2003 of the start of biological information measurement and have the smartphone 2003 provide this notification. The notification operation in this case is similar to step S807 in FIG. 12, described above.

Based on the analysis result of the atmospheric pressure, the wearable device 3501 issues an instruction to start measurement of biological information to the measurement units 201L, 201R (S3509). Measurement of biological information may instead begin in response to the user touching an instruction button, displayed on a touch panel display of the smartphone 2003, for starting biological information measurement. The wearable device 3501 may also be configured so that measurement of the biological information starts in response to user instruction or without user instruction.

After receiving the instruction to start measurement of biological information from the wearable device 3501, the measurement units 201L, 201R start measurement of biological information (S3511). The measurement units 201L, 201R measure information indicating a plethysmogram of the user in the present disclosure. The wearable device 3501 may also issue an instruction to start measurement to the measurement unit of the smartphone 2003.

The wearable device 3501 stores the biological information measured by the measurement units 201L, 201R in the storage 3515 (S3513).

After storing the measurement information, the wearable device 3501 stands by for a predetermined length of time (S3515). The wearable device 3501 stands by for two hours in step S3515. While this predetermined elapsed time is two hours in this example, the predetermined elapsed time may be any length of time other than two hours, such as five minutes, 30 minutes, one hour, six hours, one day, or the like.

After standing by for this predetermined length of time, the wearable device 3501 instructs the measurement units 201L, 201R to start measuring biological information (S3517). When the smartphone 2003 includes a measurement unit, the wearable device 3501 may also issue an instruction to start measurement to the measurement unit of the smartphone 2003.

After receiving the instruction to start measurement of biological information from the wearable device 3501, the measurement units 201L, 201R start measurement of biological information (S3519). The measurement units 201L, 201R measure information indicating a plethysmogram of the user in the present disclosure. The measurement units 201L, 201R may measure the blood flow of the user.

The wearable device 3501 stores the biological information measured by the measurement units 201L, 201R in the storage 3515 (S3521).

The wearable device 3501 compares two pieces of information related to biological information stored in the storage 3515. When a predetermined condition is satisfied, the wearable device 3501 judges whether a preventive action is to be performed (S3523). When judging that a preventive action is to be performed, the wearable device 3501 may, for example, instruct the smartphone 2003, the wearable device 3501, and the user to start a preventive action as in the above-described step S829 (S3525).

After receiving an instruction to start a preventive action from the wearable device 3501, the smartphone 2003 performs a preventive action, such as displaying an image or providing audio notification of massage information for physical condition management (S3527).

The wearable device 3501 performs a preventive action based on the instruction from the wearable device 3501 to start a preventive action (S3529). Examples of preventive actions performed by the wearable device 3501 include causing the vibration unit to vibrate and outputting predetermined audio, such as music at a Solfeggio frequency, from the vibration unit or speaker. The wearable device 3501 may perform a preventive action either in response to user instruction or, in the absence of user instruction, in response to the instruction from the wearable device 3501 to start a preventive action.

In this way, the eighth embodiment of the present disclosure achieves similar effects to the first embodiment and also allows the wearable device 3501 to analyze the atmospheric pressure and judge whether a preventive action is needed, thereby improving convenience for the user.

Ninth Embodiment Using a Body Temperature Sensor

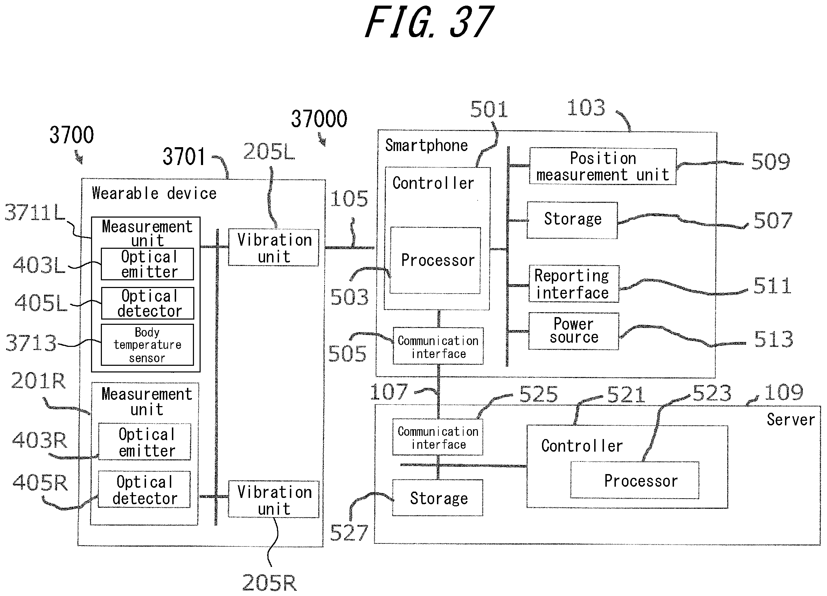

Next, a ninth embodiment of an electronic device according to the present disclosure is described with reference to FIG. 37. FIG. 37 is an internal block diagram of a physical condition management system 37000 that uses a wearable system 3700, which is an electronic device of the ninth embodiment. In the ninth embodiment, a measurement unit 3711L that corresponds to the measurement unit 201L according to the first embodiment illustrated in FIG. 5 includes a body temperature sensor 3713. In FIG. 37, the same reference numerals are used for functions and operational members that are the same as in FIG. 5. The ninth embodiment of the present disclosure is described below, focusing on the main differences from the first embodiment.

The measurement unit 3711L includes the body temperature sensor 3713 in addition to an optical emitter 403L and an optical detector 405L.

The body temperature sensor 3713 measures the body temperature of the user as biological information of the user. The body temperature measured by the body temperature sensor 3713 is stored in a storage 507 of a smartphone 103.

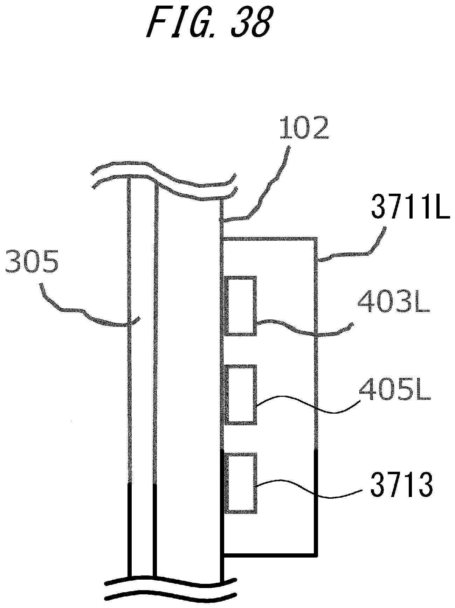

The schematic configuration of the measurement unit 3711L is described with reference to FIG. 38.

The body temperature sensor 3713 is disposed on the user side of the measurement unit 3711L to be capable of measuring the body temperature of a user 102 when a wearable device 3701 is worn by the user. The user side of the measurement unit 3711L refers to the side closer to the user when the wearable device 3701 is worn by the user.

The body temperature sensor 3713 is not limited to being installed inside the measurement unit 3711L. The body temperature sensor 3713 may be installed at any location where the body temperature of the user can be measured. For example, the body temperature sensor 3713 may be installed inside the measurement unit 201R or installed at another location in the wearable device 3701. The body temperature sensor 3713 may be installed in the smartphone 103.

Operations of the physical condition management system 37000 are nearly the same as operations of the physical condition management system 1000 illustrated in the flowchart of FIG. 12, except that the body temperature of the user is included in the biological information.

The differences from the first embodiment are therefore mainly described below with reference to FIG. 12.

After receiving the instruction to start measuring biological information from the smartphone 103, the wearable device 3701 measures the body temperature of the user along with the information indicating a plethysmogram of the user in step S811.

The wearable device 3701 transmits information also including the body temperature of the user as the measured biological information to the smartphone 103 in step S813.

In step S815, the smartphone 103 stores the biological information that includes the body temperature of the user in the storage 507.

After receiving the instruction to start measuring biological information from the smartphone 103, the wearable device 3701 measures the body temperature of the user along with the information indicating a plethysmogram of the user in step S821.

The wearable device 3701 transmits information also including the body temperature of the user as the measured biological information to the smartphone 103 in step S823.

In step S825, the smartphone 103 stores the biological information that includes the body temperature of the user in the storage 507.

In the judgment of whether a preventive action is necessary in step S827, a controller 501 of the smartphone 103 makes the judgment taking into account not only the information related to the heart rate but also the body temperature of the user at time point T1 and time point T2.

The controller 501 may provide the notification to start a preventive action when the body temperature of the user at time point T2 has fallen by more than a predetermined threshold as compared to the body temperature of the user at time point T1. The controller 501 may provide the notification to start a preventive action when the body temperature of the user at time point T2 has risen by more than a predetermined threshold as compared to the body temperature of the user at time point T1.

The controller 501 may select the preventive action to provide based not only on the information related to the heart rate but also the information of the body temperature of the user.

When outputting music to the wearable device 3701 as the preventive action, the controller 501 may instruct the wearable device 3701 to output music appropriate for the change in body temperature of the user. When the body temperature of the user has risen, the controller 501 may, for example, instruct the wearable device 3701 to output cool music such as a babbling brook. When the body temperature of the user has dropped, the controller 501 may, for example, instruct the wearable device 3701 to output music that warms the body, such as rock music.

When outputting music at a Solfeggio frequency to the wearable device 3701 as the preventive action, the controller 501 may instruct the wearable device 3701 to output music including a Solfeggio frequency appropriate for the change in body temperature of the user.

When causing the smartphone 103 to report massage information as the preventive action, the controller 501 may cause the smartphone 103 to report massage information appropriate for the change in body temperature of the user.

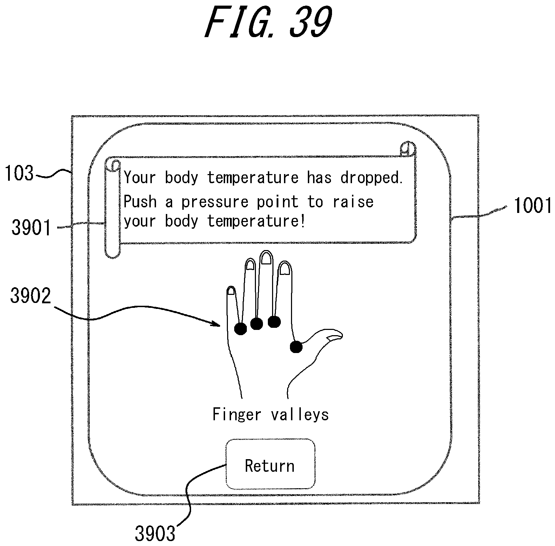

FIG. 39 illustrates an example of massage information that a touch panel display 1001 of the smartphone 103 is caused to display when the body temperature of the user has dropped. The touch panel display 1001 displays a message 3901 encouraging the user to perform a massage to raise the body temperature, an image 3902 indicating the position of an effective pressure point for raising the body temperature, and a return button 3903.

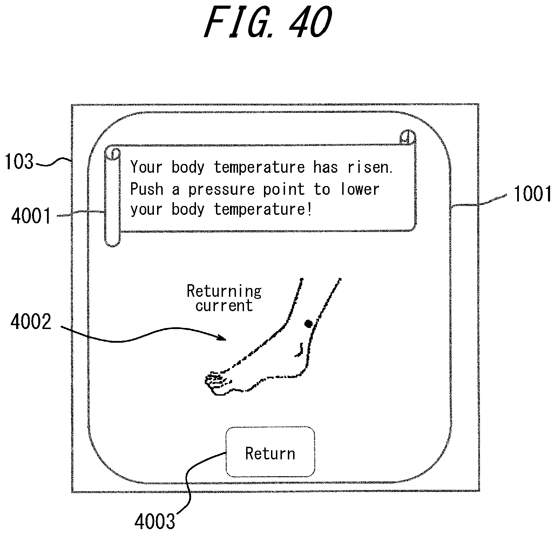

FIG. 40 illustrates an example of massage information that the touch panel display 1001 of the smartphone 103 is caused to display when the body temperature of the user has risen. The touch panel display 1001 displays a message 4001 encouraging the user to perform a massage to lower the body temperature, an image 4002 indicating the position of an effective pressure point for lowering the body temperature, and a return button 4003.

The controller 501 may select the preventive action based on information related to the heart rate and information related to the body temperature of the user, or based only on information related to the body temperature of the user.

In this way, the electronic device of the ninth embodiment also measures the body temperature of the user as biological information, allowing physical condition management that is more appropriate for the physical condition of the user.

Tenth Embodiment Using Discomfort Index

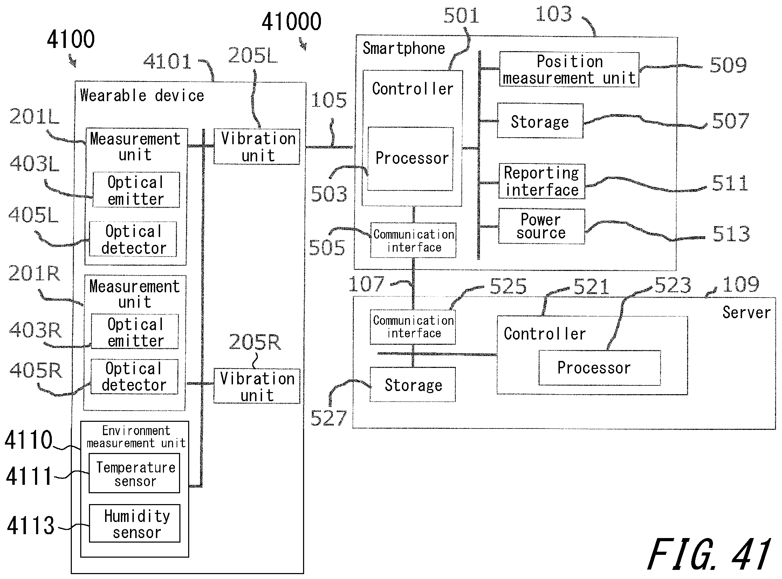

Next, a tenth embodiment of an electronic device according to the present disclosure is described with reference to FIG. 41. FIG. 41 is an internal block diagram of a physical condition management system 41000 that uses a wearable system 4100, which is an electronic device of the tenth embodiment. In the tenth embodiment, a wearable device 4101 that corresponds to the wearable device 101 according to the first embodiment illustrated in FIG. 5 includes an environment measurement unit 4110. In FIG. 41, the same reference numerals are used for functions and operational members that are the same as in FIG. 5. The tenth embodiment of the present disclosure is described below, focusing on the main differences from the first embodiment.

The wearable device 4101 includes the environment measurement unit 4110 in addition to the measurement units 201L, 201R and the vibration units 205L, 205R.

The environment measurement unit 4110 measures environment information of the environment around the user. The environment measurement unit 4110 includes a temperature sensor 4111 and a humidity sensor 4113.

The temperature sensor 4111 measures the air temperature around the user. The air temperature measured by the temperature sensor 4111 is stored in a storage 507 of a smartphone 103.

The humidity sensor 4113 measures the humidity around the user. The humidity measured by the humidity sensor 4113 is stored in the storage 507 of the smartphone 103.

A controller 501 of the smartphone 103 calculates a discomfort index DI based on the air temperature acquired from the temperature sensor 4111 and the humidity acquired from the humidity sensor 4113.

$$DI = 0.81T + 0.01H \times (0.99T - 14.3) + 46.3$$

Here, T is the air temperature [°C], and H is the humidity [%].

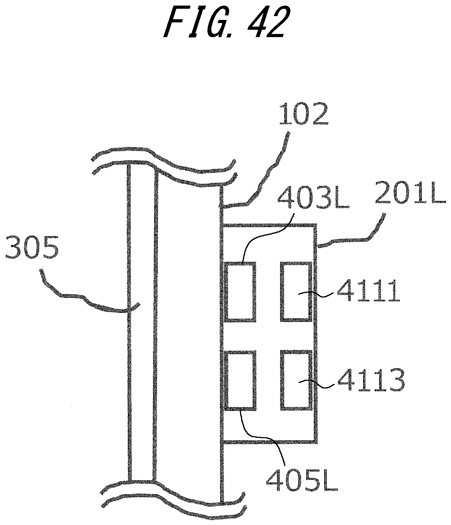

The schematic configuration when the temperature sensor 4111 and the humidity sensor 4113 are included in the measurement unit 201L is described with reference to FIG. 42.

The temperature sensor 4111 is disposed on the outside of the measurement unit 201L to be capable of measuring the air temperature around the user. Here, the outside of the measurement unit 201L refers to the side farther from the user when the wearable device 4101 is worn by the user.

The humidity sensor 4113 is disposed on the outside of the measurement unit 201L to be capable of measuring the humidity around the user.

The temperature sensor 4111 and the humidity sensor 4113 are not limited to being installed inside the measurement unit 201L. The temperature sensor 4111 may be installed at any location where the air temperature around the user can be measured. The humidity sensor 4113 may be installed at any location where the humidity around the user can be measured. For example, the temperature sensor 4111 and the humidity sensor 4113 may be installed inside the measurement unit 201R or installed at another location in the wearable device 4101. The temperature sensor 4111 and the humidity sensor 4113 may be installed in the smartphone 103.

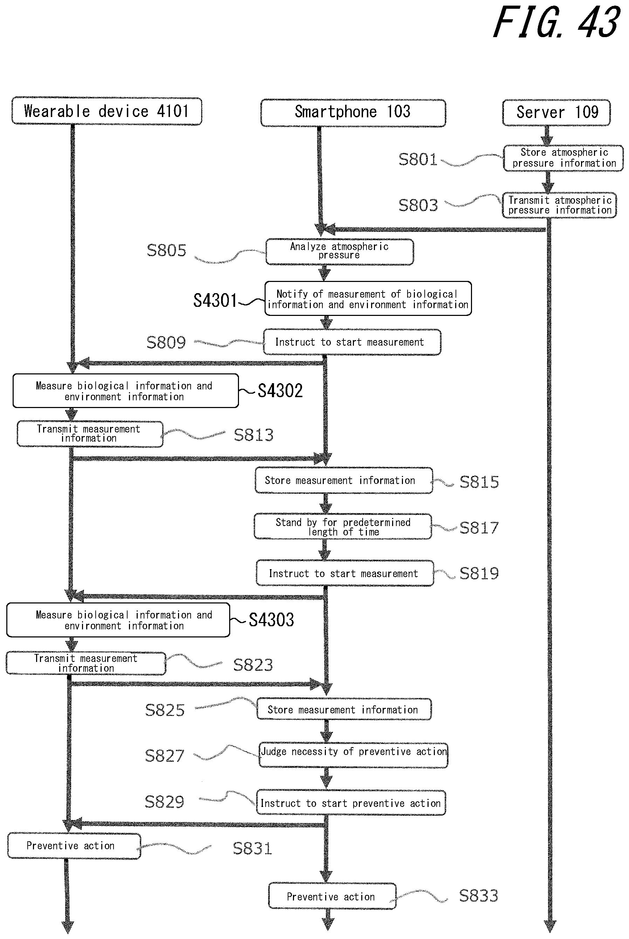

Next, with reference to the flowchart of FIG. 43, the operations of the physical condition management system 41000 illustrated in FIG. 41 are described. In FIG. 43, the same step numbers are used for operations that are similar to those of the flowchart in FIG. 12. A description of operations that are similar to the flowchart in FIG. 12 is omitted as appropriate.

Steps S801 to S805 are similar processes to steps S801 to S805 in the flowchart in FIG. 12. Hence, a description thereof is omitted.

After performing the process in step S805, the smartphone 103 provides notification to start measurement of biological information and environment information of the environment around the user based on the analysis result of the atmospheric pressure information (S4301).

Based on the analysis result of the atmospheric pressure, the smartphone 103 transmits an instruction to start measurement of biological information and environment information of the environment around the user to the wearable device 4101 (S809).

After receiving the instruction to start measurement of biological information and environment information of the environment around the user from the smartphone 103, the wearable device 4101 starts measurement of biological information and environment information of the environment around the user (S4302). The wearable device 4101 measures the air temperature around the user, using the temperature sensor 4111, as the environment information of the environment around the user. The wearable device 4101 measures the humidity around the user, using the humidity sensor 4113, as the environment information of the environment around the user.

The wearable device 4101 transmits the measured biological information and environment information of the environment around the user to the smartphone 103 (S813).

The smartphone 103 stores the biological information and environment information of the environment around the user received from the wearable device 101 in the storage 507 (S815).

The process in step S817 is the same as the process in step S817 in the flowchart of FIG. 12. Hence, a description thereof is omitted.

After standing by for a predetermined length of time, the smartphone 103 transmits an instruction to start measurement of biological information and environment information of the environment around the user to the wearable device 4101 (S819).

After receiving the instruction to start measurement of biological information from the smartphone 103, the wearable device 4101 starts measurement of biological information and environment information of the environment around the user (S4303).

The wearable device 4101 transmits the measured biological information and environment information of the environment around the user to the smartphone 103 (S823).

The smartphone 103 stores the biological information received from the wearable device 4101 in the storage 507 (S825).

In the judgment of whether a preventive action is necessary in step S827, the controller 501 of the smartphone 103 makes the judgment taking into account not only the information related to the heart rate but also the discomfort index at time point T1 and time point T2. The discomfort index is calculated by the controller 501 of the smartphone 103 based on the air temperature and humidity received as environment information from the wearable device 4101. For example, when the discomfort index at time point T2 is within a predetermined range, the controller 501 may judge that a preventive action is necessary.

The smartphone 103 provides an instruction to start the preventive action when a preventive action needs to be performed (S829). The controller 501 of the smartphone 103 may select the preventive action to provide based not only on the information related to the heart rate but also the discomfort index.

When outputting music to the wearable device 4101 as the preventive action, for example, the controller 501 may instruct the wearable device 4101 to output music corresponding to the discomfort index. When outputting music at a Solfeggio frequency to the wearable device 4101 as the preventive action, for example, the controller 501 may instruct the wearable device 4101 to output music including a Solfeggio frequency corresponding to the discomfort index.

The preventive action corresponding to the discomfort index can be stored in the storage 507 in advance based on experimental data or the like. The controller 501 may judge the effect of performing a preventive action and change the correspondence relationship between the discomfort index and the preventive action based on the result. In accordance with the result of judging the effect of performing a preventive action, the controller 501 changes the correspondence relationship, stored in the storage 507, between the discomfort index and the preventive action.

The processes in steps S831 and S833 are the same as the processes in steps S831 and S833 in the flowchart of FIG. 12. Hence, a description thereof is omitted.

The controller 501 may select the preventive action based on information related to the heart rate and the discomfort index, or based only on the discomfort index.

In this way, the electronic device of the tenth embodiment also takes into consideration the discomfort index, allowing physical condition management that is more appropriate for the physical condition of the user.

First Preventive Action

Next, a first preventive action to prevent a change in physical condition is described. The first preventive action can be applied to each of the above embodiments of the present disclosure and may be used in combination with each of the above embodiments. The first preventive action to prevent a change in physical condition uses the wearable system 100 of the first embodiment, described above. Accordingly, the first preventive action that is applicable to each of the above embodiments of the present disclosure is used in the physical condition management system 1000, to which the first embodiment of an electronic device according to the present disclosure is applied. Apart from the wearable system 100 of the first embodiment, the first preventive action to prevent a change in physical condition can also clearly be used in the electronic device of each of the above embodiments.

The first preventive action can, for example, be applied to the preventive action indicated in S831 and S833 of FIG. 12, the preventive action indicated in S3215 and S3217 of FIG. 32, the preventive action indicated in S3527 and S3529 of FIG. 36, and the like.

As described above, the parasympathetic nerves become dominant when a subject whose sympathetic nerves are dominant listens to music at a Solfeggio frequency by bone conducted vibration. The vibration units 205L, 205R mainly output human body vibration sound to the user through vibration. In the first preventive action, the vibration units 205L, 205R output music at a Solfeggio frequency to the user by bone conducted vibration. The vibration units 205L, 205R may output music at a Solfeggio frequency to the user by vibration other than bone conduction. Music at a Solfeggio frequency can also be transmitted to the user by bone conducted vibration using a speaker or the like. With bone conduction, vibration of a predetermined body part is transmitted from the bone to the auditory nerve. In the first preventive action, the vibration units 205L, 205R may output music other than music at a Solfeggio frequency to the user by bone conducted vibration.

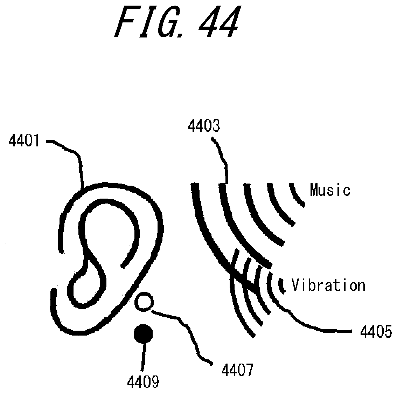

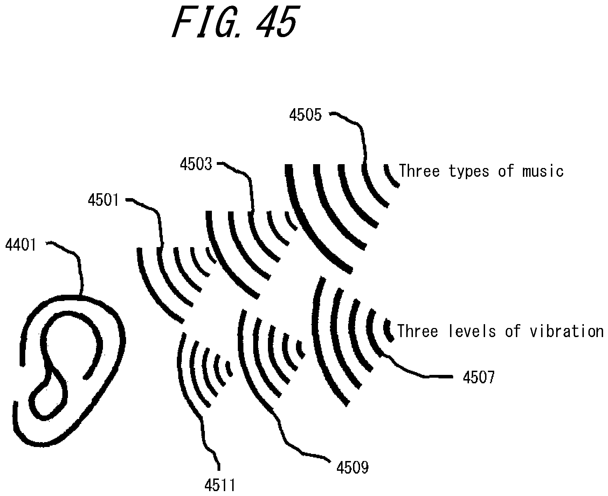

An overview of the first preventive action is provided with reference to FIG. 44 and FIG. 45. FIG. 44 and FIG. 45 are schematic views of the first preventive action.

In the first preventive action, the controller 501 controls operation of at least one of the vibration units 205L, 205R to output music 4403 and vibration 4405 to an ear 4401 of the user, as illustrated in FIG. 44.

The controller 501 controls operation of at least one of the vibration units 205L, 205R to vibrate so that the vibration 4405 stimulates the inner ear of the user. The controller 501 may control operation of at least one of the vibration units 205L, 205R to vibrate so that the vibration 4405 stimulates the vestibular nerve in the inner ear of the user. The controller 501 controls operation of at least one of the vibration units 205L, 205R to vibrate so that the vibration 4405 stimulates a pressure point of the user. Examples of the pressure points include a pressure point 4407 known as kankotsu and a pressure point 4409 known as tenyo, which is said to be effective for migraines. The pressure point stimulated by operation of at least one of the vibration units 205L, 205R may be a different pressure point.

As illustrated in FIG. 45, the controller 501 controls operation of at least one of the vibration units 205L, 205R to output three types of music, music 4501, music 4503, and music 4505 as music and three levels of vibration, vibration 4507, vibration 4509, and vibration 4511, as vibration to the ear 4401 of the user. The number of types of music is not limited to three and may be any number one or greater. The number of levels of vibration is not limited to three and may be any number one or greater. The types of music are, for example, music at a Solfeggio frequency such as water music or piano music. The types of vibration are, for example, types with different vibration intensities or frequencies. The types of music may, for example, be music other than music at a Solfeggio frequency.

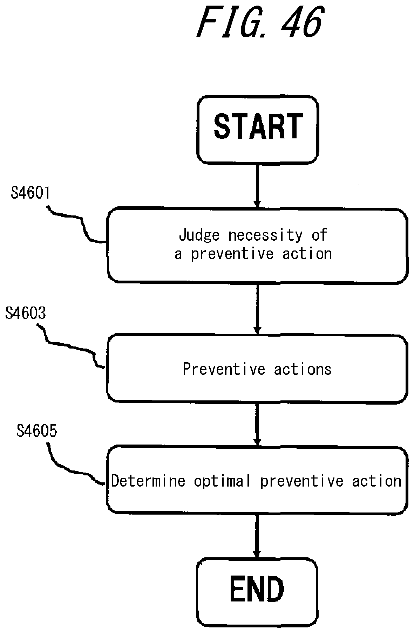

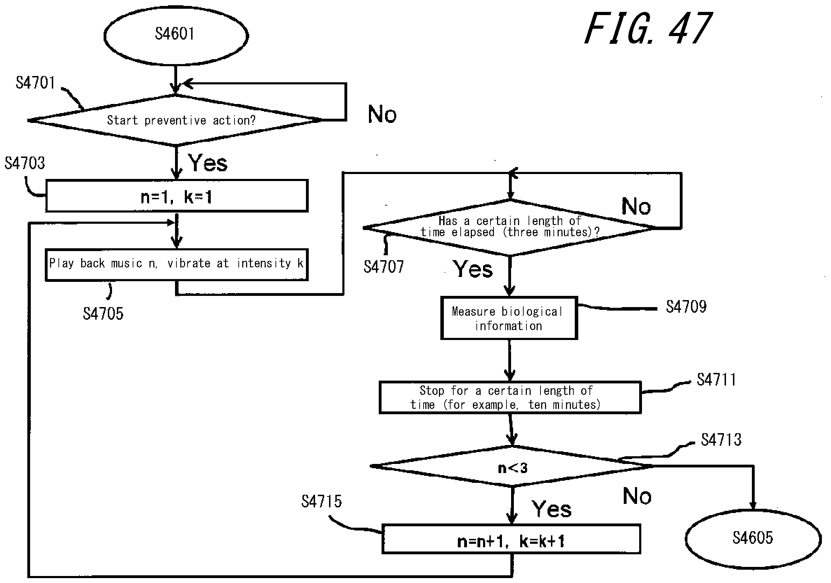

Next, the first preventive action is described with reference to FIG. 46 and FIG. 47. FIG. 46 and FIG. 47 are flowcharts of the first preventive action.

As illustrated in FIG. 46, the first preventive action mainly includes a judgment of necessity of a preventive action (S4601), preventive actions (S4603), and a determination of an optimal preventive action (S4605).

The judgment of necessity of a preventive action (S4601) is similar to the process of S827 in FIG. 12, for example.

The preventive actions (S4603) are operations illustrated in the flowchart of FIG. 47.

The determination of an optimal preventive action (S4605) is an operation to determine a preventive action that is effective for the user among the various types of preventive actions (S4603) that were performed. For example, when the LF/HF of the user is smallest after a preventive action combining music 1 with low vibration as compared to other combinations of music and vibration, the controller 501 determines that the preventive action combining music 1 with low vibration is the optimal preventive action.

The preventive actions (S4603) are described with reference to FIG. 47. Based on the result of the judgment of necessity of a preventive action (S4601) in FIG. 46, the controller 501 judges whether to start a preventive action (S4701). The controller 501 moves to step S4703 if a preventive action is to start (Yes) and stands by if not.

The controller 501 sets n to 1 and k to 1 (S4703).

The controller 501 controls operation of at least one of the vibration units 205L, 205R to play back music n and to vibrate at intensity k. Here, music n refers to the $n^{th}$ type of music. Vibration at intensity k refers to the $k^{th}$ level of vibration. The levels of vibration increase in intensity in the order of the $1^{st}$, $2^{nd}$, and $3^{rd}$ levels, for example. Instead of increasing in intensity in the order of the $1^{st}$, $2^{nd}$, and $3^{rd}$ levels, the levels of vibration may increase in intensity in a different order, or may change in any other order.

The controller 501 stands by for a certain length of time while controlling operation of at least one of the vibration units 205L, 205R to continue playing back music n and vibrating at intensity k (S4707). After this certain length of time, the controller 501 controls operation of at least one of the vibration units 205L, 205R to stop playback of music n and vibration at intensity k. The certain length of time is the time necessary to see the effect on the user of the music and vibration. This length of time is three minutes in the first preventive action but may be any length of time other than three minutes. The controller 501 moves to step S4709 after standing by for the certain length of time (Yes) and continues to stand by if the certain length of time has not elapsed (No).

The controller 501 controls operation of at least one of the measurement units 201L, 201R to measure biological information of the user (S4709). In the first preventive action, the controller 501 measures the heart rate of the user from a plethysmogram of the user, as in the first embodiment above. The controller 501 may measure the heart rate of the user from the blood flow of the user. The controller 501 measures the LF/HF from the heart rate, i.e. the measured biological information of the user.

The controller 501 stands by for a certain length of time after stopping operation of the vibration units 205L, 205R (S4711). This certain length of time is for reducing the effect on the user of playback of the music n and vibration at intensity k by operation of at least one of the vibration units 205L, 205R. This certain length of time is, for example, ten minutes but may be any length of time other than ten minutes.

The controller 501 judges whether n is less than three (S4713). The controller 501 moves to step S4715 if n is less than three (Yes) and moves to the optimal preventive action of step S4605 in FIG. 46 if n is not less than three (No).

The controller increments n and k each by one and moves to step S4705.

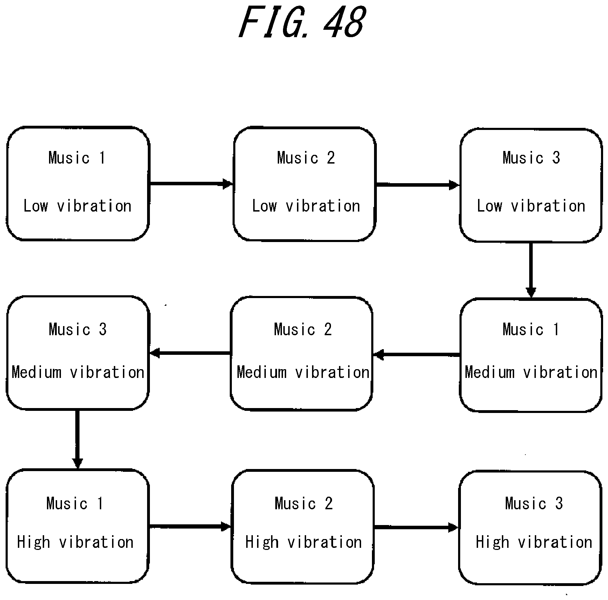

The transition of the combination of music and vibration in the flowchart of FIG. 47 is now described with reference to FIG. 48. FIG. 48 is a conceptual diagram of transitions between combinations of music and vibration during the first preventive action.

As illustrated in FIG. 48, the combination of music and vibration starts at music 1 and low vibration and transitions sequentially to music 2 and low vibration, music 3 and low vibration, ..., and music 3 and high vibration. The low vibration, medium vibration, and high vibration are types of vibration in which the vibration intensity sequentially increases.

Next, the determination of an optimal preventive action in step S4605 is described. As described above, the controller 501 changes the combination of music and vibration outputted to the user and measures the LF/HF of the user. It is assumed that at least one of the following is the case as the LF/HF decreases: the user's physical condition improves, the user relaxes, and stress decreases. Hence, the combination of music and vibration yielding the smallest LF/HF among the LF/HF values measured for the various combinations of music and vibration is determined to be the optimal preventive action.

In this way, the combination of music and vibration is sequentially changed in the first preventive action and the biological information is measured to use the LF/HF to detect the effect of the combinations of music and vibration on the user. The combination of music and vibration yielding the smallest LF/HF among the combinations of music and vibration is selected as being optimal for at least one of improving the physical condition of the user, having a relaxing effect, and reducing stress. The first preventive action therefore allows an optimal preventive action to be provided to the user.

Second Preventive Action

Next, a second preventive action to prevent a change in physical condition is described. The second preventive action can be applied to each of the above embodiments of the present disclosure and may be used in combination with each of the above embodiments. The second preventive action to prevent a change in physical condition differs from the above-described first preventive action in that instead of changing the type of music among three types, the amplitude of a predetermined frequency band of the music is sequentially changed.

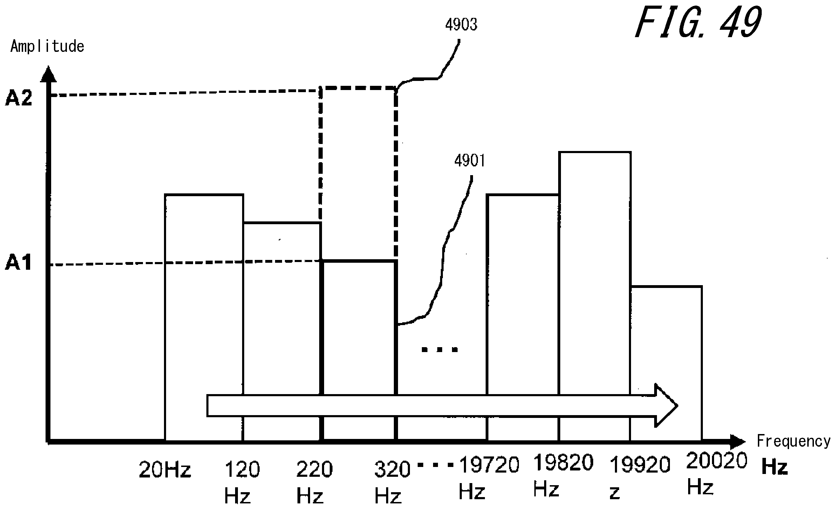

FIG. 49 is a schematic operation view of the second preventive action. As illustrated in FIG. 49, predetermined music is divided into 100 Hz bandwidths in the second preventive action. The amplitude is increased by sequentially doubling the gain in each bandwidth. The bandwidth may be any width other than 100 Hz. The bandwidth from 20 Hz to 20020 Hz is used in FIG. 49, but a bandwidth below 20 Hz or above 20020 Hz may be used. The gain is not limited to doubling and may be a predetermined gain. Instead of a gain that increases the amplitude, the gain may decrease the amplitude. In other words, when expressing the gain in decibels [dB], the gain in the second preventive action may be 0 dB or less, or may be greater than 0 dB.

As illustrated in FIG. 49, the controller 501 changes an amplitude 4901 of sound that is 220 Hz or more and less than 320 Hz among the frequencies of music to an amplitude 4903. In this case, the amplitude changes from A1 to A2, which is twice A1.

The controller 501 changes the amplitude illustrated in FIG. 49 and sequentially changes the range of the bandwidth from 20 Hz or more to less than 120 Hz, then 120 Hz or more to less than 220 Hz, etc., designating each segment of changed music as the $n^{th}$ music. When the bandwidth is 100 Hz and the frequency range of music is 20 Hz or more and less than 20020 Hz, then n is a natural number from 1 to 200.

The controller 501 designates the $n^{th}$ music as the music of the above-described first preventive action illustrated in FIG. 47 and combines the $n^{th}$ music with vibration. As in the above-described first preventive action, the controller 501 determines the optimal preventive action from among combinations of the $n^{th}$ music and vibration. The judgment of necessity of a preventive action and the determination of the optimal preventive action in the second preventive action are similar to the judgment of necessity of a preventive action (S4601) and the determination of the optimal preventive action (S4605) in the above-described first preventive action.

In this way, the second preventive action not only achieves effects similar to those of the above-described first preventive action but also sequentially changes the amplitude in the frequency band of music to create a plurality of segments of music and selects the optimal music and vibration from among combinations of these segments of music and vibration, thereby providing physical condition management that is appropriate for the user.

Experimental Method

Next, an experimental method for verifying a hypothesis of the present embodiment is described.

Hypothesis:

The parasympathetic nerves become dominant when a subject whose sympathetic nerves are dominant listens to music at a Solfeggio frequency by bone conducted vibration.

Testing method:

1. A subject with no change in physical condition such as a change in mental state occurring due to the weather, air temperature, humidity, atmospheric pressure, or other change in weather; psychosomatic pain; or the like ("change in physical condition or the like") is considered to be in a state in which the sympathetic nerves are dominant.
2. Subjects with a change in physical condition or the like are tested in an elevator to confirm validity of the hypothesis.
3. The validity of the hypothesis is confirmed with subjects who exhibited a change in physical condition or the like.
4. The validity of the hypothesis is confirmed by holding an experiential tour in a typical commercial facility where a change in atmospheric pressure can be experienced.

Conditions:

Sex, age, room without stimulation, constant air temperature/humidity, a chair such as a relaxing sofa, appropriate interior lighting, five subjects with a change in physical condition or the like, five subjects with no change in physical condition or the like, vibration frequency, vibration intensity, frequency of music, hours of sleep on test day, starting time of test (x hours after a meal), no excessive fatigue, not under excessive stress, not in poor physical condition (at least no fever).

Method of verifying effectiveness:

Measurement of autonomic nerves (change from sympathetic nerves being dominant to parasympathetic nerves being dominant), response to questions (receipt of significant responses such as comfort or alleviation of pain)

Testing method:

Change vibration intensity over three levels (in the order of low, medium, high).
Change music to three types (select a song at each of three vibration intensities).

Test in the following order:

music 1 low, music 2 low, music 3 low
music 1 medium, music 2 medium, music 3 medium
music 1 high, music 2 high, music 3 high
Provide a certain rest time between tests.
Each segment of music is at least approximately three minutes to allow measurement of autonomic nerves.

Test 1

1. Gather subjects who are not feeling pain. Measure autonomic nerves.
2. Have subjects perform calculations or the like (except for subjects who already feel a change in physical condition) for sympathetic nerves to become dominant.
3. Play music sequentially. End when confirming that parasympathetic nerves are dominant.

4. Response to questions.

Test 2

1. Gather subjects who are not feeling pain. Measure autonomic nerves.
2. Measure autonomic nerves in an elevator (high-speed if possible) used in a high-rise building.
3. After arriving at destination floor, play music sequentially. End when confirming that parasympathetic nerves are dominant.
4. Response to questions.

Test 3

1. Gather subjects who are feeling a change in physical condition or the like.
2. Measure autonomic nerve state of subjects.
3. Perform music listening test from aforementioned tests.
4. Response to questions.

Test 4 (experiment on a tour for people who feel a change in physical condition or the like to experience atmospheric pressure in the mountains)

1. Gather subjects who notice a change in physical condition or the like.
2. Measure autonomic nerve state of subjects.
3. Measure autonomic nerves while subjects experience a change in atmospheric pressure.
4. While subjects again experience a change in atmospheric pressure, measure change in autonomic nerves for music and vibration intensity that were effective in the aforementioned tests 1, 2, 3.
5. Response to questions.

Other Examples

In the present disclosure, the pulse rate is considered to be substantially equal to the heart rate.

In the present disclosure, an example of the smartphone 103 being the apparatus used in the wearable system 100 has been described. Instead of or in addition to a smartphone, however, a mobile phone, a PHS, a game device, a watch, a music player, car navigation, a television, a heart rate meter, a pulse wave monitor, a blood flow meter, any other apparatus, or any combination of these may be used in the present disclosure.

The information related to heart rate in the present disclosure refers to information from which the heart rate can be calculated. For example, this information may be the heart rate itself, the change over time in blood flow, the change over time in a plethysmogram, or the like.

In the present disclosure, the biological information may include at least one of a plethysmogram, body temperature, pulse, heart rate, pulse wave, heart rate variability, LF component of heart rate variability, HF component of heart rate variability, blood flow, blood pressure, degree of sweating, blood glucose level, and other biological information.

The case of the start of a drop in atmospheric pressure being the time point T1 for measuring the first biological information has been described in the present disclosure (see FIG. 13). The present disclosure is not, however, limited to the case of measuring the first biological information at the time point T1. Any time point related to atmospheric pressure can be used as the time point T1 for measuring the first biological information, such as when the atmospheric pressure starts to rise, or when a certain atmospheric pressure range is maintained for a predetermined time.

In the present disclosure, a device using piezoelectric elements as the vibration units 205R and 205L has been described, but the vibration unit of the present disclosure is not limited to this example. Instead of or in addition to the above-described vibration units 205R and 205L, a dynamic (electrodynamic) vibration unit, a capacitive vibration unit using a change in electrostatic energy, an electrostrictive vibration unit, a magnetic vibration unit using a change in electromagnetic energy, a carbon-type vibration unit, or the like can be used as the vibration unit of the present disclosure.

The location where biological information is measured in the present disclosure is not limited to the locations indicated in the above embodiments. The biological information of the user may be measured at a suitable location such as a finger, toe, forehead, neck, earlobe, tragus, concha auriculae, external ear canal, arm, wrist, torso, back, buttocks, waist, temple, or the like.

The location where the vibration unit applies vibration to the user for a massage may be a suitable location such as a finger, toe, forehead, neck, earlobe, tragus, concha auriculae, external ear canal, arm, wrist, torso, back, buttocks, waist, temple, or the like.

The autonomic nerves control certain organs of the body. The autonomic nerves are constituted by two nervous systems, the sympathetic nervous system and the parasympathetic nervous system. Tense sympathetic nerves may be considered a stressed state, whereas tense parasympathetic nerves may be considered a relaxed state.

Audio is outputted using a piezoelectric element in the present disclosure. Instead of or in addition to a piezoelectric element, however, a speaker or the like can be used to output audio.

Biological information has been described as being measured in response to the measurement instruction from the user in the present disclosure. When the measurement unit of the electronic device is in contact with a living body, however, measurement of biological information may start automatically. In this case, the wearable device may include a living body detection sensor, such as an illumination sensor for detecting contact with a living body.

A time point T2 for measuring biological information in the present disclosure occurs after a predetermined time elapses from a drop in atmospheric pressure, or when the atmospheric pressure becomes equal to or less than a predetermined atmospheric pressure threshold Pt. The time point T2 may, however, occur when the atmospheric pressure lowers from the atmospheric pressure at time point T1 by a predetermined amount. For example, in the example illustrated in FIG. 13, the time point at which the atmospheric pressure drops by 5 [hPa] from 1003 [hPa] at time point T1 to 998 [hPa] may be taken as the time point T2 for measuring the biological information. In this case, a preventive action becomes possible due to the change in atmospheric pressure, with no need to wait for the predetermined time to elapse. The physical condition can thus be managed appropriately. The predetermined drop in atmospheric pressure is not limited to 5 [hPa] and may be any value.

In the present disclosure, an example of the biological information measurement sensor being a reflective plethysmograph or blood flow meter has been described. The biological information sensor of the present disclosure may instead be a transmission-type plethysmograph or blood flow meter.

In the present disclosure, an atmospheric pressure sensor may be provided in an apparatus other than the smartphone 103, such as the wearable device 101.

In the present disclosure, a measurement apparatus such as a temperature gauge, humidity gauge, or altitude gauge may be provided in the wearable device 101, the smartphone 103, or the like. A change in the physical condition of the user can be detected more appropriately based on information such as the temperature, humidity, and altitude measured in this way. In other words, the information such as the temperature, humidity, and altitude measured in this way may be compared with thresholds and used as factors for judging a change in the physical condition of the user.

In the present disclosure, the server 109 may notify the smartphone 103 of the timing for measuring biological information based on atmospheric pressure information stored in the storage 527. The smartphone 103 may then report the measurement of biological information based on this notification. The operation for the server 109 to calculate the timing at which the smartphone 103 is to measure biological information based on atmospheric pressure information stored in the storage 527 is similar to the operation described using the examples in FIG. 12, FIG. 13, or other examples in the above embodiments. In this case, the server 109 can calculate the timing for measuring biological information and provide notification, thereby simplifying the processing on the smartphone 103.

In the present disclosure, the server 109 may receive the biological information from the smartphone 103 and transmit an instruction to execute a preventive action to the smartphone 103 based on the biological information. The smartphone 103 may then perform a preventive action based on the instruction. The operations for the server 109 to receive the biological information from the smartphone 103 and transmit an instruction to execute a preventive action to the smartphone 103 based on the biological information are similar to the above-described operations of step S827 and the like of FIG. 12. In this case, the server 109 can transmit an instruction to execute a preventive action, thereby simplifying the processing on the smartphone 103.

When the wearable device, the smartphone, or the server acquires atmospheric pressure information in the present disclosure, the atmospheric pressure information may be acquired from 1) the server of an atmospheric pressure observation system, such as the Automated Meteorological Data Acquisition System, which is a regional weather observation system operated by the Japan Meteorological Agency, 2) a terminal, such as a smartphone, tablet, or mobile phone, with a built-in atmospheric pressure sensor, 3) an atmospheric pressure sensor provided in the actual wearable device, such as an earphone, headphone, headset, or head-up display, or from any combination of the above.

The atmospheric pressure sensor in the present disclosure may optionally be provided in any one or more of the wearable device, the smartphone, the server, and other apparatuses. The atmospheric pressure sensor may instead be provided in only one of the wearable device, the smartphone, the server, or another apparatus.

The configurations of the embodiments of the present disclosure, the technical configurations of elements of the embodiments, the operations of the embodiments, the technical operations of elements of the embodiments, the configurations of modifications, the configurations of additional examples, the configurations of other examples, the operations of modifications, the operations of additional examples, the operations of other examples, and the like may be freely selected and combined as an electronic device, server, data structure, physical condition management method, and physical condition management program of the present disclosure.

REFERENCE SIGNS LIST

| 100 | Wearable system |
|---|---|
| 101 | Wearable device |
| 102 | User |
| 102E | Ear of user |
| 103 | Smartphone |
| 105 | Cable |
| 107 | Network |
| 109 | Server |
| 201L, 201R | Measurement unit |
| 203L, 203R | Holding portion |
| 205L, 205R | Vibration unit |
| 207 | Connector |
| 305 | Superficial temporal artery |
| 307 | Internal region below skin surface |
| 309 | Vestibular nerve |
| 403L, 403R | Optical emitter |
| 405L, 405R | Optical detector |
| 501 | Controller |
| 503 | Processor |
| 505 | Communication interface |
| 507 | Storage |
| 509 | Position measurement unit |
| 511 | Reporting interface |
| 513 | Power source |
| 521 | Controller |
| 523 | Processor |
| 525 | Communication interface |
| 527 | Storage |
| 1000 | Physical condition management system |
| 1601 | Piezoelectric element |
| 1603 | Panel |
| 1801L, | 1801R Measurement unit |
| 1803L | Optical emitter |
| 1805L | Optical detector |
| 2001 | Wearable system |
| 2003 | Smartphone |
| 2005 | Atmospheric pressure sensor |
| 2201 | Smartphone |
| 2203 | Optical detector |
| 2205 | Optical emitter |
| 2301 | Finger of user |
| 2303 | Blood vessel of finger of user |
| 2401 | Measurement unit |
| 2410 | Wearable system |
| 2601 | Wearable system |
| 2603 | Wearable device |
| 2603L | Left-ear earphone |
| 2603R | Right-ear earphone |
| 2605L | Outer-ear insertion portion |
| 3501 | Wearable device |
| 3503 | Wearable system |

| 3511 | Controller |
| 3513 | Processor |
| 3515 | Storage |
| 3517 | Position measurement unit |
| 3519 | Atmospheric pressure sensor |
| 3700 | Wearable system |
| 3701 | Wearable device |
| 3711L | Measurement unit |
| 3713 | Body temperature sensor |
| 4100 | Wearable system |
| 4101 | Wearable device |
| 4110 | Environment measurement unit |
| 4111 | Temperature sensor |
| 4113 | Humidity sensor |
| 20000 | Physical condition management system |
| 24000 | Physical condition management system |
| 35000 | Physical condition management system |
| 37000 | Physical condition management system |
| 41000 | Physical condition management system |

## Claims

1. An electronic device comprising:

   a measurement unit configured to measure biological information of a user; and
   a controller configured to perform a predetermined preventive action based on the biological information measured by the measurement unit and atmospheric pressure information.

2. The electronic device of claim 1, further comprising a first reporting interface configured to report a start of measurement of the biological information based on the atmospheric pressure information.

3. The electronic device of claim 1, further comprising a vibration unit configured to provide the user with a vibration as the preventive action.

4. The electronic device of claim 3, wherein the vibration includes vibration that stimulates a vestibular nerve.

5. The electronic device of claim 1, further comprising an output interface configured to output audio as the preventive action.

6. The electronic device of claim 5, wherein the audio is music combined with sound at a Solfeggio frequency.

7. The electronic device of claim 1, further comprising a second reporting interface configured to report massage information as the preventive action.

8. The electronic device of claim 3, further comprising:

   a holding portion configured to be held by an auricle of an ear of the user;
   wherein the vibration unit is disposed in the holding portion at a back head side of the ear; and
   wherein the measurement unit is disposed in the holding portion at a face side of the ear.

9. The electronic device of claim 3, further comprising a storage configured to store the atmospheric pressure information.

10. The electronic device of claim 9, further comprising:

    a main unit comprising the controller and the storage;
    wherein information is transmitted from the main unit to the vibration unit by wired communication or wireless communication.

11. The electronic device of claim 1, wherein the atmospheric pressure information is information acquired over a network.

12. The electronic device of claim 1, further comprising:

an atmospheric pressure sensor configured to measure atmospheric pressure;
wherein the atmospheric pressure information is information measured by the atmospheric pressure sensor.

13. The electronic device of claim 12, wherein the electronic device comprises a smartphone, and the atmospheric pressure sensor is disposed in the smartphone.

14. The electronic device of claim 12, wherein the electronic device comprises a wearable device to be worn by the user, and the atmospheric pressure sensor is disposed in the wearable device.

15. The electronic device of claim 1,
wherein the biological information measured when a decrease in the atmospheric pressure begins is designated as first biological information;
wherein the biological information measured after a predetermined time elapses from when the decrease begins is designated as second biological information; and
wherein the controller performs the preventive action on the user based on a comparison between a value related to the first biological information and a value related to the second biological information.

16. The electronic device of claim 1,
wherein the biological information measured when the atmospheric pressure becomes a predetermined value or less is designated as third biological information; and
wherein the controller performs the preventive action on the user based on a value related to the third biological information.

17. The electronic device of claim 15,
wherein the controller measures information related to a heart rate as the biological information;
wherein the controller calculates a power spectrum density from heart rate variability, which is variation in intervals of the heart rate; and
wherein the controller calculates the value related to the first biological information and the value related to the second biological information based on a value related to an LF component, which is a low-frequency region, and a value related to an HF component, which is a high-frequency region.

18. The electronic device of claim 17, wherein the value related to the first biological information and the value related to the second biological information are each a value yielded by dividing the value related to the LF component by the value related to the HF component.

19. The electronic device of claim 16,
wherein the controller measures information related to a heart rate as the biological information;
wherein the controller calculates a power spectrum density from heart rate variability, which is variation in intervals of the heart rate; and
wherein the controller calculates the value related to the third biological information based on a value related to an LF component, which is a low-frequency region, and a value related to an HF component, which is a high-frequency region.

20. The electronic device of claim 19, wherein the value related to the third biological information is a value yielded by dividing the value related to the LF component by the value related to the HF component.

21. The electronic device of claim 17,
wherein the low-frequency region is a region of 0.05 Hz or more to less than 0.15 Hz in the power spectrum density; and
wherein the high-frequency region is a region of 0.15 Hz or more to less than 0.40 Hz in the power spectrum density.

22. The electronic device of claim 19,
wherein the low-frequency region is a region of 0.05 Hz or more to less than 0.15 Hz in the power spectrum density; and

wherein the high-frequency region is a region of 0.15 Hz or more to less than 0.40 Hz in the power spectrum density.

23. The electronic device of claim 1, wherein the measurement unit is a plethysmograph configured to measure a value related to a plethysmogram, the plethysmograph comprising an optical emitter configured to emit light and an optical detector configured to detect light.

24. The electronic device of claim 1, wherein the measurement unit is a blood flow meter configured to measure a value related to blood flow, the blood flow meter comprising an optical emitter configured to emit light and an optical detector configured to detect light.

25. The electronic device of claim 1,
wherein a server connected to the electronic device over a network provides the electronic device with a notification of timing for measurement of biological information based on the atmospheric pressure information; and
wherein the electronic device is configured to report measurement of biological information based on the notification.

26. The electronic device of claim 1,
wherein a server connected to the electronic device over a network receives the biological information from the electronic device and transmits an instruction to execute the preventive action to the electronic device based on the biological information; and
wherein the electronic device is configured to perform the preventive action based on the instruction.

27. The electronic device of claim 15,
wherein the controller is configured to cause the measurement unit to measure fourth biological information after the preventive action; and
wherein the controller performs the preventive action on the user based on a comparison between the value related to the second biological information and a value related to the fourth biological information.

28. The electronic device of claim 1,
wherein the biological information measured when a decrease in the atmospheric pressure begins is designated as first biological information;
wherein the biological information measured when the atmospheric pressure decreases by a predetermined amount from the atmospheric pressure when the first biological information was measured is designated as second biological information; and
wherein the controller performs the preventive action on the user based on a comparison between a value related to the first biological information and a value related to the second biological information.

29. The electronic device of claim 1, wherein the measurement unit is configured to measure a body temperature of the user as the biological information.

30. The electronic device of claim 1, wherein the measurement unit is configured to measure a body temperature of the user and information related to a heart rate as the biological information.

31. The electronic device of claim 1, further comprising:

an environment measurement unit configured to measure environment information of an environment around the user;
wherein the controller is configured to perform the predetermined preventive action based on the biological information, the environment information, and the atmospheric pressure information.

32. The electronic device of claim 31,
wherein the environment information includes air temperature information and humidity information;
wherein the controller is configured to calculate a discomfort index based on the air temperature information and the humidity information; and
wherein the controller is configured to perform the predetermined preventive action based on the biological information, the discomfort index, and the atmospheric pressure information.

33. An electronic device comprising:

an environment measurement unit configured to measure environment information of an environment around a user; and

a controller configured to perform a predetermined preventive action based on the environment information and atmospheric pressure information.

34. A server connected over a network to the electronic device of any one of claims 1 to 33;
   wherein the server is configured to receive the atmospheric pressure information from the electronic device and provide the electronic device with a notification of timing for measurement of biological information based on the atmospheric pressure information; and
   wherein the electronic device is configured to report measurement of biological information based on the notification.

35. A server connected over a network to the electronic device of any one of claims 1 to 33;
   wherein the server is configured to receive the biological information from the electronic device and transmit an instruction to execute the preventive action to the electronic device based on the biological information; and
   wherein the electronic device is configured to perform the preventive action based on the instruction.

36. A data structure comprising:

   biological information related to a heart rate of a user; and
   information related to atmospheric pressure;
   wherein the data structure is configured to cause an electronic device to

   acquire the information related to the heart rate of the user based on the information related to atmospheric pressure; and
   perform a predetermined preventive action based on the information related to the heart rate.

37. A physical condition management method comprising; measuring biological information of a user; and performing a predetermined preventive action based on the measured biological information and atmospheric pressure information.

38. A physical condition management program for causing a computer to:

   measure biological information of a user; and
   perform a predetermined preventive action based on the measured biological information and atmospheric pressure information.

39. The electronic device of claim 1,
   wherein the controller comprises:

   a vibration unit configured to provide the user with a vibration as the preventive action; and
   an output interface configured to output audio as the preventive action;

   wherein the controller changes a combination of a type of the vibration and a type of the audio; and
   wherein the controller selects a combination of the type of the vibration and the type of the audio based on a measurement result of the biological information measured when the combination of the type of the vibration and the type of the audio was changed.

## FIG. 1

FIG. 2

EP 3 646 780 A1

# FIG. 3

EP 3 646 780 A1

# FIG. 4

102

201L

305

403L

405L

FIG. 5

Wearable device 100 / 101

- Measurement unit 201L
  - Optical emitter 403L
  - Optical detector 405L
- Vibration unit 205L
- Measurement unit 201R
  - Optical emitter 403R
  - Optical detector 405R
- Vibration unit 205R

1000

Smartphone 103

- Controller 501
  - Processor 503
- Communication interface 505
- Position measurement unit 509
- Storage 507
- Reporting interface 511
- Power source 513

105

107

Server 109

- Communication interface 525
- Controller 521
  - Processor 523
- Storage 527

# FIG. 6

Location A

| Time | Atmospheric pressure (hPa) |
|---|---|
| 8:00 | 1000 |
| 9:00 | 1003 |
| 10:00 | 1002 |
| 11:00 | 1001 |
| 12:00 | 1000 |
| 13:00 | 999 |
| 14:00 | 999 |
| 15:00 | 998 |
| 16:00 | 999 |

*FIG. 7*

# FIG. 8

Storage 507

| | |
|---|---|
| User ID | 7100 |
| Telephone number | 7103 |
| Atmospheric pressure information | 7106 |
| Biological information | 7109 |
| Position information | 7112 |
| Vibration pattern | 7115 |
| Massage information | 7118 |
| Music information | 7121 |
| History of preventive actions | 7124 |

# FIG. 9

9120

9121          9123   9125

| Date and time | Location | Atmospheric pressure information |
|---|---|---|

# FIG. 10

10130

10131    10133    10135

| User ID | Date and time | Information related to heartbeat (LF/HF) |

# FIG. 11

Storage 527

| | |
|---|---|
| User ID | 7200 |
| Telephone number | 7203 |
| Atmospheric pressure information | 7206 |
| Biological information | 7209 |
| Position information | 7212 |
| Vibration pattern | 7215 |
| Massage information | 7218 |
| Music information | 7221 |
| History of preventive actions | 7224 |

## FIG. 12

| Wearable device 101 | Smartphone 103 | Server 109 |
|---|---|---|

S801 — Store atmospheric pressure information

S803 — Transmit atmospheric pressure information

S805 — Analyze atmospheric pressure

S807 — Notify regarding measurement of biological information

S809 — Instruct to start measurement

Measure biological information — S811

Transmit measurement information — S813

Store measurement information — S815

Stand by for predetermined length of time — S817

Instruct to start measurement — S819

Measure biological information — S821

Transmit measurement information — S823

S825 — Store measurement information

S827 — Judge necessity of preventive action

S829 — Instruct to start preventive action

Preventive action — S831

Preventive action — S833

# FIG. 13

Change in atmospheric pressure at location A

# FIG. 14

103

1001

1003

Atmospheric pressure
starting to drop!

Measure physical condition!

1007

Return

Start measurement

1005

# FIG. 15

Judgment of necessity of
preventive action

Extract heart rate fluctuation from
first pulse wave, calculate first
power spectrum density of
heart rate fluctuation — S1101

Calculate first LF/HF value
from first power spectrum density — S1103

A

# FIG. 16

A

Extract heart rate fluctuation from second pulse wave, calculate second power spectrum density of heart rate fluctuation

S1105

Calculate second LF/HF value from second power spectrum density

S1107

B

# FIG. 17

B

Compare first LF/HF value and second LF/HF value,
judge whether second LF/HF value is
greater than first LF/HF value

S1109

Instruct to start preventive action if second LF/HF
value is greater than first LF/HF value

S1111

# FIG. 18

1 0 3

1 4 0 1

Physical condition has changed.
Perform next preventive action!

1001

1 4 0 3 — Start vibration

1 4 0 5 — Massage

1 4 0 7 — Music

1 4 0 9 — Return

# FIG. 19

103 ～

1101

1501

1503

1505

1507

Massage #1

Up-down movement of ear

Lightly pull ear up and
down, left and right

Return

1509

## FIG. 20

```
┌─────────────────────────────────────┐
│ Vibration unit 205L                  │
│                                      │
│   ┌───────────────────┐              │
│   │   Piezoelectric   │───────  1 6 0 1
│   │     element       │              │
│   └───────────────────┘              │
│                                      │
│   ┌───────────────────┐              │
│   │                   │───────  1 6 0 3
│   │      Panel        │              │
│   └───────────────────┘              │
│                                      │
└─────────────────────────────────────┘
```

# FIG. 21

# FIG. 22

# FIG. 23

# FIG. 24

**Smartphone** (2003)

- Position measurement unit (509)
- Atmospheric pressure sensor (507)
- Storage (2005)
- Reporting interface (511)
- Power source (513)
- Controller (501)
  - Processor
- Communication interface (503, 505)

**Server** (109)

- Controller (523)
  - Processor (521)
- Communication interface (525)
- Storage (527)

**Wearable device** (101, 2001)

- Vibration unit (205R)
- Measurement unit (201L)
  - Optical emitter (403L)
  - Optical detector (405L)
- Measurement unit (201R)
  - Optical emitter (403R)
  - Optical detector (405R)
- Vibration unit (205L)

(20000, 105, 107)

FIG. 25

Change in atmospheric pressure at location A

# FIG. 26

2201

2203

2205

EP 3 646 780 A1

## FIG. 27

# FIG. 28

# FIG. 29

| Wearable device 101 | Smartphone 2201 | Server 109 |
|---|---|---|

S801 — Store atmospheric pressure information

S803 — Transmit atmospheric pressure information

S805 — Analyze atmospheric pressure

S807 — Notify regarding measurement of biological information

S2501 — Instruct to start measurement

S2503 — Measure biological information

S2505 — Store biological information

Stand by for predetermined length of time — S817

S2507 — Instruct to start measurement

S2509 — Measure biological information

S2511 — Store biological information

S827 — Judge necessity of preventive action

S829 — Instruct to start preventive action

Preventive action

S831

Preventive action — S833

# FIG. 30

# FIG. 31

405L

403L

2603L

2605L

205L

105

# FIG. 32

| Wearable device 101 | Smartphone 103 |
|---|---|

Notify of completion ～ S3201

S3203 ～ Instruct to start measurement

Measure biological information ～ S3205

Transmit biological information ～ S3207

S3209 ～ Store biological information

S3211 ～ Judge effect

S3213 ～ Instruct to start preventive action

Preventive action

S3215

S3217 ～ Preventive action

# FIG. 33

| Frequency | Power |
|---|---|
| 0 | 102.9124 |
| 0.015625 | 2.553626 |
| 0.03125 | 1.302153 |
| 0.046875 | 1.690787 |
| 0.0625 | 2.851957 |
| 0.078125 | 1.152959 |
| 0.09375 | 2.204631 |
| 0.109375 | 0.868121 |
| 0.125 | 1.534859 |
| 0.140625 | 1.74404 |

| Frequency | Power |
|---|---|
| 0.15625 | 1.225979 |
| 0.171875 | 1.352217 |
| 0.1875 | 0.663797 |
| 0.203125 | 1.073383 |
| 0.21875 | 0.278176 |
| 0.234375 | 0.590297 |
| 0.25 | 0.381906 |
| 0.265625 | 0.910864 |
| 0.28125 | 0.788615 |
| 0.296875 | 0.589352 |

| Frequency | Power |
|---|---|
| 0.3125 | 1.247238 |
| 0.328125 | 1.568714 |
| 0.34375 | 0.99026 |
| 0.359375 | 1.30191 |
| 0.375 | 1.961401 |
| 0.390625 | 1.371537 |
| 0.40625 | 1.057826 |
| 0.421875 | 1.429651 |
| 0.4375 | 1.122276 |
| 0.453125 | 2.076937 |

| Frequency | Power |
|---|---|
| 0.46875 | 1.89779 |
| 0.484375 | 1.957828 |
| 0.5 | 1.898315 |
| 0.515625 | 1.634152 |
| 0.53125 | 1.140689 |
| 0.546875 | 1.263673 |
| 0.5625 | 1.770602 |
| 0.578125 | 1.083971 |
| 0.59375 | 1.177132 |
| 0.609375 | 1.274233 |

| Frequency | Power |
|---|---|
| 0.625 | 1.299779 |
| 0.640625 | 1.420689 |
| 0.65625 | 0.382396 |
| 0.671875 | 0.670719 |
| 0.6875 | 0.631357 |
| 0.703125 | 0.495559 |
| 0.71875 | 1.134539 |
| 0.734375 | 0.466334 |
| 0.75 | 0.505505 |
| 0.765625 | 1.082286 |

| Frequency | Power |
|---|---|
| 0.78125 | 1.397868 |
| 0.796875 | 1.289288 |
| 0.8125 | 0.450433 |
| 0.828125 | 1.142429 |
| 0.84375 | 0.819077 |
| 0.859375 | 0.91162 |
| 0.875 | 1.109824 |
| 0.890625 | 2.557588 |
| 0.90625 | 0.34936 |
| 0.921875 | 0.812306 |

| Frequency | Power |
|---|---|
| 0.9375 | 0.780733 |
| 0.953125 | 1.027532 |
| 0.96875 | 1.510006 |
| 0.984375 | 0.589434 |
| 1 | 1.85817 |

FIG. 34

# FIG. 35

## FIG. 36

| Smartphone 2003 | Wearable device 3501 | Server 109 |
|---|---|---|

S3501 — Store atmospheric pressure information

S3503 — Transmit atmospheric pressure information

S3505 — Analyze atmospheric pressure

S3507 — Notify regarding measurement of biological information

S3509 — Instruct to start measurement

S3511 — Measure biological information

S3513 — Store biological information

S3515 — Stand by for predetermined length of time

S3517 — Instruct to start measurement

S3519 — Measure biological information

S3521 — Store biological information

S3523 — Judge necessity of preventive action

S3525 — Instruct to start preventive action

Preventive action

S3527

Preventive action — S3529

FIG. 37

EP 3 646 780 A1

# FIG. 38

## FIG. 39

103

1001

3901

Your body temperature has dropped.
Push a pressure point to raise
your body temperature!

3902

Finger valleys

Return

3903

# FIG. 40

103

4001 — Your body temperature has risen.
Push a pressure point to lower
your body temperature!

1001

Returning
current

4002

Return

4003

FIG. 41

# FIG. 42

*FIG. 43*

| Wearable device 4101 | Smartphone 103 | Server 109 |
|---|---|---|

S801 — Store atmospheric pressure information

S803 — Transmit atmospheric pressure information

S805 — Analyze atmospheric pressure

S4301 — Notify of measurement of biological information and environment information

S809 — Instruct to start measurement

Measure biological information and environment information ~ S4302

Transmit measurement information — S813

Store measurement information ~ S815

Stand by for predetermined length of time ~ S817

Instruct to start measurement ~ S819

Measure biological information and environment information ~ S4303

Transmit measurement information — S823

S825 — Store measurement information

S827 — Judge necessity of preventive action

S829 — Instruct to start preventive action

Preventive action — S831

Preventive action — S833

# FIG. 44

# FIG. 45

4505

4503

4501

4401

Three types of music

Three levels of vibration

4507

4509

4511

# FIG. 46

```
          ┌─────────────┐
          │    START    │
          └──────┬──────┘
                 │
                 ▼
S4601    ┌───────────────────────┐
         │   Judge necessity of  │
         │  a preventive action  │
         └───────────┬───────────┘
                     │
                     ▼
S4603    ┌───────────────────────┐
         │   Preventive actions  │
         └───────────┬───────────┘
                     │
                     ▼
S4605  ┌─────────────────────────────────┐
       │ Determine optimal preventive action │
       └─────────────────┬───────────────┘
                         │
                         ▼
                ┌─────────────┐
                │     END     │
                └─────────────┘
```

# FIG. 47

S4601

S4701 — Start preventive action? — No

Yes

S4703 — n=1, k=1

S4705 — Play back music n, vibrate at intensity k

S4707 — Has a certain length of time elapsed (three minutes)? — No

Yes

S4709 — Measure biological information

S4711 — Stop for a certain length of time (for example, ten minutes)

S4713 — n<3 — No

Yes

S4715 — n=n+1, k=k+1

S4605

# FIG. 48

```
┌─────────────┐      ┌─────────────┐      ┌─────────────┐
│   Music 1   │ ───▶ │   Music 2   │ ───▶ │   Music 3   │
│ Low vibration│      │ Low vibration│      │ Low vibration│
└─────────────┘      └─────────────┘      └──────┬──────┘
                                                  │
                                                  ▼
┌─────────────┐      ┌─────────────┐      ┌─────────────┐
│   Music 3   │ ◀─── │   Music 2   │ ◀─── │   Music 1   │
│Medium vibration│    │Medium vibration│    │Medium vibration│
└──────┬──────┘      └─────────────┘      └─────────────┘
       │
       ▼
┌─────────────┐      ┌─────────────┐      ┌─────────────┐
│   Music 1   │ ───▶ │   Music 2   │ ───▶ │   Music 3   │
│High vibration│      │High vibration│      │High vibration│
└─────────────┘      └─────────────┘      └─────────────┘
```

FIG. 49

## INTERNATIONAL SEARCH REPORT

| International application No. |
| --- |
| PCT/JP2018/021388 |

### A.  CLASSIFICATION OF SUBJECT MATTER
Int.Cl. A61B5/02(2006.01)i, H04R1/00(2006.01)i, H04R17/00(2006.01)i

According to International Patent Classification (IPC) or to both national classification and IPC

### B.  FIELDS SEARCHED

Minimum documentation searched (classification system followed by classification symbols)
Int.Cl. A61B5/00-5/22, H04R1/00, H04R17/00

| Documentation searched other than minimum documentation to the extent that such documents are included in the fields searched | |
| --- | --- |
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

### C.  DOCUMENTS CONSIDERED TO BE RELEVANT

| Category* | Citation of document, with indication, where appropriate, of the relevant passages | Relevant to claim No. |
| --- | --- | --- |
| X | JP 2016-158768 A (PIONEER CORP.) 05 September 2016, paragraphs [0012]-[0034], fig. 1, 2 (Family: none) | 1-3, 5, 9-16, 23-32, 34-38 |
| Y | | 17-22 |
| A | | 4, 6-8, 39 |

☒  Further documents are listed in the continuation of Box C.       ☐  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15 August 2018 (15.08.2018) | 28 August 2018 (28.08.2018) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2018/021388

C (Continuation).　DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2008-148948 A (TERUMO CORP.) 03 July 2008, paragraphs [0006]-[0034], [0043]-[0076] (Family: none) | 1-2, 5-6, 11-16, 23-32, 34-38<br>17-22, 39<br>3-4, 7-10 |
| X<br>Y<br>A | JP 2017-33301 A (NTT SOFTWARE CORP.) 09 February 2017, paragraphs [0010]-[0013], [0033]-[0043] (Family: none) | 1-3, 7, 9-16, 23-32, 34-38<br>4, 8, 17-22, 39<br>5-6 |
| Y | JP 2017-38911 A (TANIGUCHI, Kazuhiro) 23 February 2017, paragraphs [0017], [0025], [0071]-[0073], [0099] (Family: none) | 4, 8 |
| Y | US 2010/0186577 A1 (KIM et al.) 29 July 2010, paragraphs [0022]-[0065] & KR 10-2010-0086618 A | 17-22 |

Form PCT/ISA/210 (continuation of second sheet) (January 2015)

## INTERNATIONAL SEARCH REPORT

| International application No. |
|---|
| PCT/JP2018/021388 |

**Box No. II        Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: 1-32, 34-39

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

| International application No. |
|---|
| PCT/JP2018/021388 |

<Continuation of Box III>

(Invention 1) Claims 1-32 and 34-39 (excluding portions referring to claim 33 in claims 34 and 35)

Document 1 cited in the partial international search report indicates that biometric information is acquired on the basis of atmospheric pressure information, and altering (corresponding to the "preventive action" of the present application) is performed by processing the information. Claim 1 lacks novelty in light of document 1, and thus does not have a special technical feature. Furthermore, claim 1 lacks novelty in light of documents 2 and 3.

Claim 2, which is dependent on claim 1, has a special technical feature of a notification unit for notifying initiation of measurement of biometric information. Furthermore, claims 3-32 and 34-39 are dependent on claim 1 or substantially identical to a main invention. Therefore, claims 1-32 and 34-39 are classified as invention 1.

(Invention 2) Claim 33 and claims 34 and 35 referring to claim 33

Claim 33 is not considered to share identical or corresponding technical features with claim 2 classified as invention 1.

Furthermore, claim 33 is not dependent on claim 1 classified as invention 1.

Furthermore, claim 33 is not substantially identical or equivalent to any of the claims classified as invention 1.

Therefore, claim 33 cannot be classified as invention 1.

Claim 33 has a special technical feature of an "electronic device" which "performs a prescribed preventive operation on the basis of environment information and atmospheric pressure information", and is thus classified as invention 2.

Form PCT/ISA/210 (extra sheet) (January 2015)

## REFERENCES CITED IN THE DESCRIPTION

**Patent documents cited in the description**

- JP 2017125505 A
- JP 2017138398 A
- JP 2017151907 A

- JP 2017181820 A
- JP 2016055155 A

**Non-patent literature cited in the description**

- **SATO, JUN.** Heal weather-related pain and say goodbye to headaches, vertigo, and stress!. Fusosha Publishing Inc, 01 November 2015, 21-23, 33-37, 112-114